# EUROPEAN PATENT APPLICATION

(11) **EP 4 831 154 A1**
(43) Date of publication of application: **28.10.2026**
(21) Application number: 25742110.7
(22) Date of filing: 03.01.2025
(51) Int. Cl.: C07C 211/61, C07D 307/91, C07D 333/76, C07D 209/88, C07D 409/12, H10K 85/60

(54) **COMPOUND FOR ORGANIC ELECTRIC ELEMENT, ORGANIC ELECTRIC ELEMENT USING SAME, AND ELECTRONIC DEVICE COMPRISING SAME**

(30) Priority: 18.01.2024 KR 20240008083
(71) Applicant: Duk San Neolux Co., Ltd., Chungcheongnam-do 31027 (KR)
(72) Inventor: KANG, Moon Sung, Cheonan-si Chungcheongnam-do 31027 (KR); PARK, Sang Yong, Cheonan-si Chungcheongnam-do 31027 (KR); LEE, Jung Geun, Cheonan-si Chungcheongnam-do 31027 (KR)
(74) Representative: Prüfer & Partner mbB Patentanwälte · Rechtsanwälte
(86) International application number: PCT/KR2025/000135
(87) International publication number: WO 2025/155011

(57) **Abstract**

Provided are a compound represented by Formula 1, a method for recovering the compound, an organic electronic device including a first electrode, a second electrode, and an organic layer between the first electrode and the second electrode, and an electronic apparatus comprising the device, wherein the compound of Formula 1 is comprised in the organic layer of the device, thereby the driving voltage of the organic electronic device can be lowered, and the luminous efficiency and lifetime of the device can be improved.

## Description

### TECHNICAL FIELD

The present invention relates to a compound for an organic electronic device, an organic electronic device using the same, and an electronic apparatus thereof.

### BACKGROUND

In general, organic electroluminescence refers to a phenomenon in which electronical energy is converted into light energy by an organic material. An organic electronic device utilizing organic electroluminescence typically includes an anode, a cathode, and an organic layer interposed therebetween. In many cases, the organic layer has a multilayered structure comprising different materials, respectively, in order to improve the efficiency and stability of an organic electronic device. For example, the organic layer may comprise a hole injection layer, a hole transport layer, an emission layer, an electron transport layer, and an electron injection layer.

The most significant issues associated with organic electroluminescent devices are lifetime and efficiency. As display panels increase in size, it has become imperative to resolve these issues related to lifetime and efficiency.

Efficiency, lifetime, driving voltage, and the like are interrelated. An increase in efficiency may lead to a decrease in driving voltage, which in turn may reduce the crystallization of organic materials caused by Joule heating generated during device operation. As a result, the lifetime of the device may be extended.

However, simply improving the organic layers does not maximize efficiency. This is because long lifetime and high efficiency can be simultaneously achieved only when the energy levels and T₁ values between respective organic layers, as well as the intrinsic properties of the materials (e.g., mobility, interfacial characteristics, etc.), are optimally combined.

Therefore, in order to fully realize the excellent characteristics of organic electronic device, it is necessary to develop materials constituting the organic layers within the device, particularly an emission auxiliary layer material.

### Detailed Description of the Invention

### Technical Challenge

An object of the present invention is to provide a compound for an organic electronic device capable of lowering the driving voltage of the device and improving luminous efficiency and lifetime of the device, an organic electronic device using the same, and an electronic apparatus thereof.

### Means of Solving Problems

In an aspect of the present invention, the present invention provides a compound represented by the following Formula.

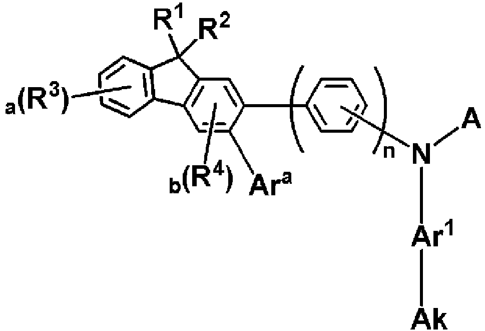

In another aspect, the present invention provides an organic electronic device comprising a compound represented by the above formula, and an electronic apparatus comprising the same.

### Effect of Invention

By using the compound according to an embodiment of the present invention as a material for an organic electronic device, it is possible to lower the driving voltage of the device and improve luminous efficiency and lifetime.

### BRIEF DESCRIPTION OF THE DRAWINGS

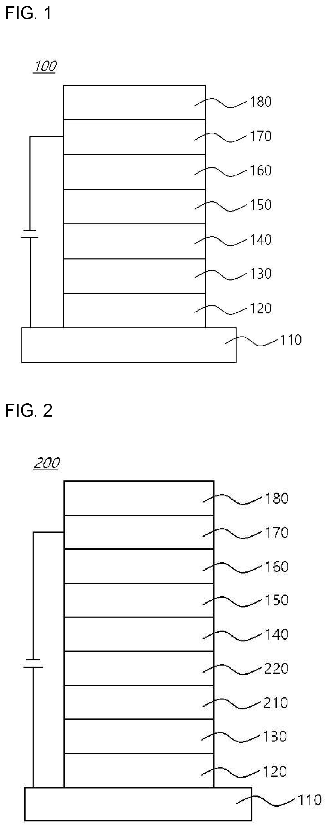

Figures 1 to 3 illustrate an example of organic electroluminescent device according to the present invention.

### [Description of Reference Numerals]

| | | | |
|---|---|---|---|
| 100, 200, 300: | organic electronic device | 110: | first electrode |
| 120: | hole injection layer | 130: | hole transport layer |
| 140: | emission layer | 150: | electron transport layer |
| 160: | electron injection layer | 170: | second electrode |
| 180: | layer for improving light efficiency | 210: | buffer layer |
| 220: | emission auxiliary layer | 320: | first hole injection layer |
| 330: | first hole transport layer | 340: | first emission layer |
| 350: | first electron transport layer | 360: | first charge-generation layer |
| 361: | second charge-generation layer | 420: | second hole injection layer |
| 430: | second hole transport layer | 440: | second emission layer |
| 450: | second electron transport layer | CGL: | charge generation layer |
| ST1: | first stack | ST2: | second stack |

### DETAILED DESCRIPTION

As used herein, the terms "aryl group," "arylene group," and "aromatic ring" refer to a hydrocarbon aromatic ring group, and unless otherwise specified, each has 6 to 60 carbon atoms, without being limited thereto. In the present invention, the aryl group or arylene group includes monocyclic, polycyclic, and fused ring structures.

As used herein, the term "fluorenyl group" refers to a substituted or unsubstituted fluorenyl group, and the term "fluorenylene group" refers to a substituted or unsubstituted fluorenylene group. The fluorenyl group or fluorenylene group used in the present invention includes spiro compounds formed by the bonding of R and R' in the following structure, and also includes compounds in which adjacent R" groups are bonded to form a ring. The terms "substituted fluorenyl group" and "substituted fluorenylene group" mean that at least one of R, R', or R" in the following structure is a substituent other than hydrogen. In the following structure, the number of R" groups may range from 1 to 8. Throughout this specification, the fluorenyl group and fluorenylene group may collectively be referred to as a "fluorene group" or "fluorene," regardless of their valence.

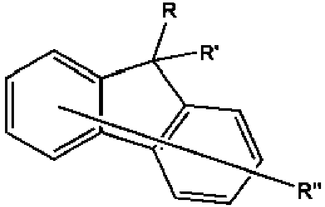

As used herein, the term "spiro compound" refers to a compound having a "spiro linkage," wherein the spiro linkage means a connection in which two rings share only one atom. The atom shared by the two rings is referred to as a "spiro atom," and depending on the number of spiro atoms contained in a compound, such compounds are referred to as "monospiro-," "dispiro-," or "trispiro-" compounds, respectively.

As used herein, the term "heterocyclic group" comprises both aromatic rings, such as a "heteroaryl group" or a "heteroarylene group," and non-aromatic rings. Unless otherwise specified, the "heterocyclic group" refers to a ring structure containing one or more heteroatoms and having from 2 to 60 carbon atoms, but is not limited thereto. The term "heteroatom," as used herein, refers to atoms such as nitrogen (N), oxygen (O), sulfur (S), phosphorus (P), or silicon (Si), and may also include heteroatomic groups such as SO₂, P=O, and the like, which can replace a carbon atom in the ring structure as shown in the following compound.

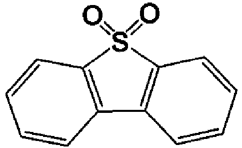

In addition, the heterocyclic group includes monocyclic, polycyclic, or condensed(fused) rings containing a heteroatom. In the case of condensed rings, if at least one of the rings in the condensed system contains a heteroatom, it is defined as a heterocycle. For example, the condensed ring systems where a heterocycle such as furan, dihydrofuran, thiophene, pyrrole, or pyridine is fused with an aromatic ring such as benzene, naphthalene, or phenanthrene, or with an alicyclic ring such as cyclopentane or cyclohexane fall under the category of heterocycles. Likewise, a spiro compound in which at least one ring contains a heteroatom is also considered heterocycle.

As used herein, the term "alicyclic group" refers to a cyclic hydrocarbon group excluding aromatic hydrocarbons, and complies monocyclic, polycyclic, fused ring, and spiro compounds, and unless otherwise specified, refers to a ring having 3 to 60 carbon atoms, without being limited thereto. In particular, in the present specification, an alicyclic ring (group) is defined as a hydrocarbon ring containing no aromatic ring. Accordingly, not only saturated hydrocarbon rings such as cycloalkyl groups, but also rings having one or more double bonds within the ring are regarded as alicyclic rings, so long as they are not aromatic hydrocarbons.

The term "a fused ring (group)" or "a condensed ring (group)" as used in the present specification refers, unless otherwise specified, to a ring formed by the fusion of an alicyclic ring and an aromatic hydrocarbon ring (aromatic ring or aryl ring), and, unless otherwise stated, it means a ring condensed of an alicyclic ring having 3 to 60 carbon atoms and an aromatic hydrocarbon having 6 to 60 carbon atoms.

In this specification, the 'group name' corresponding to an aryl group, an arylene group, a heterocyclic group, and the like, exemplified for each symbol and its substituent, may be expressed either as 'a functional group name reflecting the valence' or as 'the name of a parent compound'. For example, in the case of 'phenanthrene,' which is a type of aryl group, it may be described as 'phenanthryl' when referring to a monovalent group, and as 'phenanthrylene' when referring to a divalent group. Alternatively, it may also be described by its parent compound name 'phenanthrene,' regardless of valence. Similarly, in the case of pyrimidine, it may be referred to as 'pyrimidine' regardless of its valence. Alternatively, it may be described by the name of the corresponding functional group, such as 'pyrimidinyl' for a monovalent group and 'pyrimidinylene' for a divalent group.

In addition, in the present specification, numerical and alphabetical indicators of positions may be omitted when describing the name of a compound or a substituent. For example, compounds such as pyrido[4,3-d]pyrimidine, benzofuro[2,3-d]pyrimidine, and 9,9-dimethyl-9H-fluorene may be described in a simplified manner as pyridopyrimidine, benzofurropyrimidine, and dimethylfluorene, respectively. Accordingly, both benzo[g]quinoxaline and benzo[f]quinoxaline may be generally referred to as benzoquinoxaline.

In addition, unless otherwise specified, the definitions of substituents in the Formula used in the present invention may be applied in accordance with the definitions of the index in the following Formula.

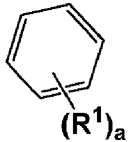

Here, when "a" is an integer of 0, it means that the substituent R¹ is absent. In other words, when a is 0, all carbon atoms forming the benzene ring are bonded to hydrogen atoms, and in this case, the hydrogen atoms bonded to the carbon atoms may be omitted in the depiction of the formula or compound. In addition, when "a" is an integer of 1, one substituent R¹ is bonded to any one of the carbon atoms forming the benzene ring. When "a" is an integer of 2 or 3, the substituents may be bonded as shown below, and when "a" is an integer from 4 to 6, the substituents are also bonded to the carbon atoms of the benzene ring in a similar manner. When "a" is an integer of 2 or more, the R¹ substituents may be the same or different from each other.

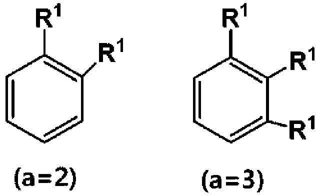

In addition, unless otherwise specified, the term "ring" as used in this specification refers to an aryl ring, a heteroaryl ring, a fluorene ring, an alicyclic ring, a fused ring, and the like. The expression "number-ring" denotes a fused(condensed) ring system, whereas "number-membered ring" may refer to the shape of the ring. For example, naphthalene corresponds to a fused ring consisting of two rings, anthracene corresponds to a fused ring consisting of three rings, thiophene and furan correspond to five-membered heterocycles, and benzene and pyridine corresponds to six-membered aromatic rings.

In addition, unless otherwise specified in the present specification, when adjacent groups are linked to each other to form a ring, the ring may be selected from the group consisting of a C₆-C₆₀ aromatic ring group; a fluorenyl group; a C₂-C₆₀ heterocyclic group containing at least one heteroatom selected from O, N, S, Si, and P; a C₃-C₆₀ alicyclic group; and a fused ring of a C₃-C₆₀ alicyclic ring and a C₆-C₆₀ aromatic ring. Here, the aromatic ring group may be an aryl ring, and the heterocyclic group may comprise a heteroaryl ring.

Unless otherwise specified, the term 'adjacent groups,' as used herein, comprises not only the relationships such as R₁ and R₂, R₂ and R₃, R₃ and R₄, and R₅ and R₆ but also R₇ and R₈ sharing a common carbon atom. It may further comprise cases substituents attached to different ring-forming atoms (e.g., carbon or nitrogen), such as R₁ and R₇, R₁ and R₈, or R₄ and R₅. That is, even when substituents are not directly adjacent on the same atom, one substituent may be considered adjacent to another substituent attached to a neighboring ring-forming atom. Additionally, substituents bonded to the same carbon atom forming the ring may also be regarded as adjacent groups. In the following Formula, when substituents such as R₇ and R₈, which are bonded to the same carbon atom, are connected to form a ring, a compound containing a spiro moiety may be formed.

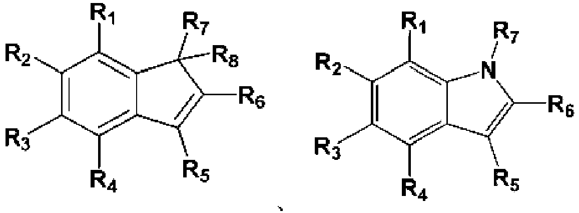

In addition, in the present specification, the expression 'adjacent groups may be linked to each other to form a ring' is used in the same sense as 'adjacent groups are selectively linked to each other to form a ring,' and refers to a case where at least one pair of adjacent groups may be bonded to form a ring.

In addition, unless otherwise specified in the present specification, substituents such as an aryl group, an arylene group, a fluorenyl group, a fluorenylene group, a heterocyclic group, an alicyclic group, a fused ring, an alkyl group, an alkenyl group, an alkynyl group, an alkoxyl group, an aryloxyl group, alkylthio group, arylthio group, etc., and a ring formed by adjacent groups may be each substituted with one or more substituents selected from the group consisting of deuterium; halogen; a cyano group; a nitro group; siloxane group; a C₆-C₃₀ aryl group; a fluorenyl group; a C₂-C₃₀ heterocyclic group containing at least one heteroatom of O, N, S, Si and P; a C₃-C₃₀ alicyclic group; a fused ring group of a C₃-C₆₀ alicyclic ring and a C₆-C₆₀ aromatic ring; a C₁-C₂₀ alkyl group; a C₂-C₂₀ alkenyl group; a C₂-C₂₀ alkynyl group; a C₁-C₂₀ alkoxyl group; a C₆-C₂₀ aryloxy group; a C₁-C₂₀ alkylthio group; a C₆-C₂₀ arylthio group; a silane group unsubstituted or substituted with a C₁-C₂₀ alkyl group or a C₆-C₂₀ aryl group; and a phosphine oxide group unsubstituted or substituted with a C₁-C₂₀ alkyl group or a C₆-C₂₀ aryl group.

Unless otherwise specified herein, the symbol "*" or " 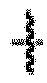 " represents a bonding site.

Hereinafter, with reference to Figures 1 to 3, the laminated structure of an organic electronic device comprising the compound according to the present invention will be described.

In assigning reference numerals to the constituent elements in the drawings, it should be noted that like reference numerals are used to designate like constituent elements throughout the drawings, wherever possible, even though the constituent elements are illustrated in different drawings. In addition, in describing the present invention, detailed descriptions of related well-known configurations or functions will be omitted when they are deemed to unnecessarily obscure the gist of the present invention.

In describing the constituent elements of the present invention, terms such as first, second, A, B, (a), and (b) may be used. These terms are used solely for the purpose of distinguishing one constituent element from another and are not intended to limit the nature, order, or sequence of the corresponding constituent elements. When a constituent element is described as being "linked," "bonded," or "connected" to another constituent element, it should be understood that the constituent element may be directly linked, bonded, or connected to the other constituent element, or that one or more additional constituent elements may be interposed therebetween.

Further, when a constituent element, such as a layer, film, region, or substrate, is described as being "on" or "over" another constituent element, it should be understood that the constituent element may be directly on the other constituent element or that one or more additional constituent elements may be interposed therebetween. Conversely, when a constituent element is described as being "directly on" another constituent element, it should be understood that no intervening constituent element is present therebetween.

Figures 1 to 3 respectively illustrate examples of an organic electronic device according to embodiments of the present invention.

Referring to FIG. 1, an organic electronic device 100 according to an embodiment of the present invention comprises a first electrode 110, a second electrode 170, and an organic layer formed between the first electrode 110 and the second electrode 170 on a substrate (not shown), and an inorganic layer may also be comprised between the first electrode 110 and the second electrode 120.

For example, the first electrode 110 may be an anode (positive electrode), and the second electrode 170 may be a cathode (negative electrode). In an inverted organic electronic device, however, the first electrode may be a cathode, while the second electrode may be an anode.

The organic layer refers to a layer comprising at least one organic material. For example, the organic layer may include a hole injection layer 120, a hole transport layer 130, an emission layer 140, an electron transport layer 150, and an electron injection layer 160. Further, the electron injection layer 160 may be an inorganic layer that does not contain any organic material.

Specifically, a hole injection layer 120, a hole transport layer 130, an emission layer 140, an electron transport layer 150, and an electron injection layer 160 may be sequentially formed on the first electrode 110.

Preferably, a layer for improving the luminous efficiency 180 may be formed on one side of either the first electrode 110 or the second electrode 170, which does not face the organic layer or the inorganic layer. When the layer for improving the luminous efficiency 180 is formed, the luminous efficiency of the organic electronic device can be enhanced.

For example, when the layer for improving the luminous efficiency 180 may be formed on the second electrode 170, in the case of a top-emission organic electroluminescent device, optical energy loss due to surface plasmon polaritons (SPPs) at the second electrode 170 may be reduced, and in the case of a bottom-emission organic electroluminescent device, the layer for improving the luminous efficiency 180 may function as a buffer layer for the second electrode 170.

A buffer layer 210 or an emission auxiliary layer 220 may additionally be formed between the hole transport layer 130 and the emission layer 140, as will be described with reference to FIG. 2.

Referring to FIG. 2, an organic electronic device 200 according to another embodiment of the present invention may sequentially include a hole injection layer 120, a hole transport layer 130, a buffer layer 210, an emission auxiliary layer 220, an emission layer 140, an electron transport layer 150, an electron injection layer 160, and a second electrode 170 on a first electrode 110, and a layer for improving the luminous efficiency 180 may be formed on the second electrode.

Although not illustrated in FIG. 2, an electron transport auxiliary layer may additionally be formed between the emission layer 140 and the electron transport layer 150.

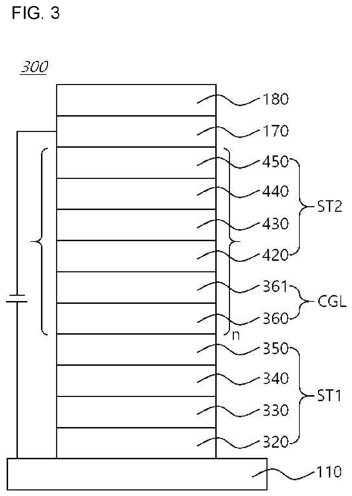

In addition, according to another embodiment of the present invention, an organic layer may be in the form of a plurality of stacks, each including a hole transport layer, an emission layer, and an electron transport layer. This will be described with reference to FIG. 3.

Referring to FIG. 3, an organic electronic device 300 according to another embodiment of the present invention may include two or more sets of stacks (ST1, ST2) of organic layers formed in multiple layers between the first electrode 110 and the second electrode 170, and a charge generation layer (CGL) may be formed between the stacks of the organic layers.

Specifically, the organic electronic device according to the embodiment of the present invention may comprise a first electrode 110, a first stack ST1, a charge generation layer CGL, a second stack ST2, and a second electrode 170 and a layer for improving light efficiency 180.

The first stack ST1 is an organic layer formed on the first electrode 110, and may comprise a first hole injection layer 320, a first hole transport layer 330, a first emission layer 340, and a first electron transport layer 350. The second stack ST2 may comprise a second hole injection layer 420, a second hole transport layer 430, a second emission layer 440, and a second electron transport layer 450. As such, the first stack and the second stack may have the same or different stacked structures of organic layers.

The charge generation layer CGL may be formed between the first stack ST1 and the second stack ST2. The charge generation layer CGL may comprise a first charge generation layer 360 and a second charge generation layer 361. It is formed between the first emission layer 340 and the second emission layer 440 to enhance the current efficiency of each emission layer and facilitate charge distribution.

The first emission layer 340 may comprise an emissive material that comprises a blue host doped with a blue fluorescent dopant, and the second emission layer 440 may comprise an emissive material that comprises a green host doped with both a greenish-yellow dopant and a red dopant. However, the materials of the first emission layer 340 and the second emission layer 440 according to an embodiment of the present invention are not limited thereto.

In FIG. 3, n may be an integer from 1 to 5, and when n is 2, a charge generation layer (CGL) and a third stack may be additionally formed on the second stack ST2.

When a plurality of emission layers are formed in a multilayer stack structure as shown in FIG. 3, it is possible to manufacture an organic electroluminescent device that emits not only white light but also various colors, where the white light is produced by the mixing of light emitted from each emission layer.

Compound represented by Formula 1 of the present invention may be comprised in an organic layer. For example, the compound represented by Formula 1 of the present invention can be used as a material for a hole injection layer 120, 320, 420, a hole transport layer 130, 330, 430, a buffer layer 210, an emission auxiliary layer 220, an electron transport layer 150, 350, 450, an emission layer 140, 340, 440, and/or a light efficiency improving layer 180, preferably, as the material of an emission auxiliary layer 220.

Even when a similar core structure is used, the band gap, electrical properties, interfacial properties, and the like may vary depending on the position at which substituents are introduced and the type of substituents bonded thereto. Accordingly, studies on the selection of the core and combinations of sub-substituents bonded thereto are required. In particular, when the energy levels and T₁ values between respective organic layers, as well as intrinsic material properties (e.g., mobility, interfacial properties, etc.), form an optimized combination, both long lifetime and high efficiency can be achieved simultaneously.

Therefore, by using the compound represented by Formula 1 as the material of an emission auxiliary layer 220, it is possible to optimize the energy levels, T₁ values between organic layers, and intrinsic material properties (e.g., mobility, interfacial properties, etc.), thereby simultaneously improving the lifetime and efficiency of the organic electronic device.

An organic electroluminescent device according to an embodiment of the present invention may be manufactured using various deposition methods. It may be fabricated using deposition methods such as physical vapor deposition (PVD) or chemical vapor deposition (CVD). For example, a metal, a conductive metal oxide, or an alloy thereof may be deposited on a substrate to form an anode 110, and an organic layer including a hole injection layer 120, a hole transport layer 130, an emission layer 140, an electron transport layer 150, and an electron injection layer 160 may be formed thereon, followed by deposition of a material usable as a cathode 170 thereon. In addition, an emission-auxiliary layer 220 may be further formed between the hole transport layer 130 and the emission layer 140, and an electron transport auxiliary layer (not shown) may be further formed between the emission layer 140 and the electron transport layer 150, or a stacked structure may be formed as described above.

In addition, the organic layer may be formed using various polymer materials by a solution process or solvent process rather than a deposition method, for example, a spin coating process, nozzle printing process, inkjet printing process, slot coating process, dip coating process, roll-to-roll process, doctor blading process, screen printing process, or thermal transfer method, and may be manufactured with a reduced number of layers. Since the organic layer according to the present invention may be formed by various methods, the scope of the present invention is not limited by the formation method thereof.

An organic electronic device according to an embodiment of the present invention may be a top emission type, bottom emission type, or dual emission type depending on the materials used.

In addition, the organic electronic device according to an embodiment of the present invention may be selected from the group consisting of an organic electroluminescent device, an organic solar cell, an organic photoreceptor, an organic transistor, a monochromatic lighting device, and a quantum dot display device.

Another embodiment of the present invention may include a display device including the organic electronic device described above, and an electronic apparatus including a control unit for controlling the display device. In this case, the electronic apparatus may be a current or future wired or wireless communication terminal, and may include all electronic devices such as mobile communication terminals including mobile phones, navigation devices, game consoles, various televisions, and computers.

Hereinafter, a compound according to one aspect of the present invention will be described.

A compound according to one aspect of the present invention is represented by Formula 1 below.

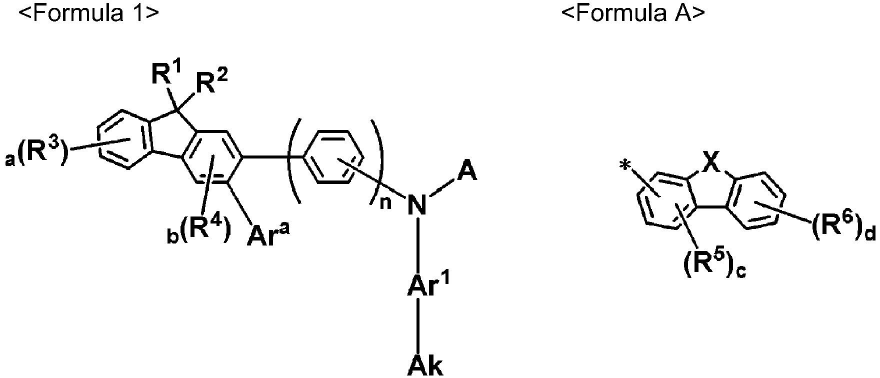

In the above Formula, each symbol is defined as follows.

A is Formula A, and X is C(R^{a})(R^{b}), O, S or N(R^{c}).

Ak is selected from the group consisting of Formula Ak-1 to Formula Ak-3.

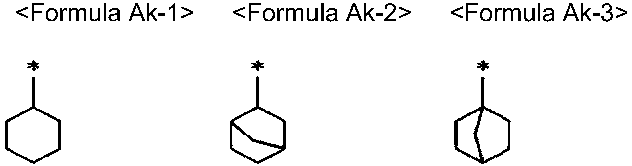

In Formula Ak-1 to Ak-3, hydrogen may be replaced with deuterium.

Ar¹ is selected from the group consisting of a C₆-C₆₀ arylene group; a fluorenylene group; a C₂-C₆₀ heterocyclic group containing at least one heteroatom of O, N, S, Si and P; a fused ring of a C₆-C₆₀ aromatic ring and a C₃-C₆₀ alicyclic group; and a combination thereof. Herein, the combination thereof may be, for example, a combination of an arylene group and an arylene group, a combination of an arylene group and a fluorenylene group, a combination of an arylene group and a heterocyclic group, a combination of a fluorenylene group and a heterocyclic group, a combination of a heterocyclic group and a heterocyclic group, or the like.

Ar^{a} is a C₆-C₆₀ aryl group or a fluorenyl group.

R¹ and R² is a C₁-C₂₀ alkyl group, wherein the hydrogen of the alkyl group may be replaced with deuterium, provided that R¹ and R² do not form a ring together.

5 R³ to R⁶ are each independently selected from the group consisting of hydrogen; deuterium; halogen; a cyano group; a nitro group; a silane group; a C₆-C₆₀ aryl group; a fluorenyl group; a C₂-C₆₀ heterocyclic group containing at least one heteroatom selected from O, N, S, Si, and P; a C₃-C₆₀ alicyclic group; a fused ring group of a C₆-C₆₀ aromatic ring and a C₃-C₆₀ alicyclic ring; a C₁-C₂₀ alkyl group; a C₂-C₂₀ alkenyl group; a C₂-C₂₀ alkynyl group; a C₁-C₂₀ alkoxy group; and a C₆-C₆₀ aryloxy group, and adjacent groups may be bonded to each other to form a ring.

a and d are each an integer of 0 to 4, b is an integer of 0 to 2, c is an integer of 0 to 3, n is an integer of 0 or 1, and when any one of a to d is an integer of 2 or more, the plurality of R³, the plurality of R⁴, the plurality of R⁵, and the plurality of R⁶ may be the same as or different from each other.

When adjacent groups, for example, adjacent R³ groups, adjacent R⁴ groups, adjacent R⁵ groups, adjacent R⁶ groups are be bonded to each other to form a ring, the ring is selected from the group consisting of a C₆-C₆₀ aromatic ring group; a fluorenyl group; a C₃-C₆₀ alicyclic group; a C₂-C₆₀ heterocyclic group containing at least one heteroatom of O, N, S, Si and P; and a fused ring of a C₃-C₆₀ alicyclic group and a C₆-C₆₀ aromatic ring.

When adjacent groups are bonded to each other to form an aromatic ring, the aromatic ring may be, for example, a C₆-C₂₀, C₆-C₁₈, C₆-C₁₆, C₆-C₁₄, C₆-C₁₃, C₆-C₁₂, C₆-C₁₀, C₆, C₁₀, C₁₂, C₁₄, C₁₅, C₁₆, or C₁₈ aromatic ring, and may specifically be benzene, naphthalene, anthracene, phenanthrene, pyrene, triphenylene, or the like.

n is an integer of 0 or 1. When n is 0, the amino group is directly bonded to the benzene ring of fluorene, and when n is 1, the amino group is bonded to the benzene ring of fluorene through a phenylene linker.

R^{a} and R^{b} are each independently selected from the group consisting of hydrogen; deuterium; halogen; a cyano group; a nitro group; a C₆-C₆₀ aryl group; a fluorenyl group; a C₂-C₆₀ heterocyclic group containing at least one heteroatom selected from O, N, S, Si, and P; a C₃-C₆₀ alicyclic group; a fused ring group of a C₆-C₆₀ aromatic ring and a C₃-C₆₀ alicyclic ring; a C₁-C₂₀ alkyl group; a C₂-C₂₀ alkenyl group; a C₂-C₂₀ alkynyl group; a C₁-C₂₀ alkoxy group; and a C₆-C₆₀ aryloxy group. With the proviso that the case where R^{a} and R^{b} are linked to each other to form a ring is excluded.

R^{c} is selected from the group consisting of a C₆-C₆₀ aryl group; a fluorenyl group; a C₂-C₆₀ heterocyclic group containing at least one heteroatom of O, N, S, Si and P; and a fused ring of a C₆-C₆₀ aromatic ring and a C₃-C₆₀ alicyclic group.

When at least one of Ar^{a}, R³ to R⁶, R^{a} to R^{c} is an aryl group, or Ar¹ is an arylene group, the aryl group or arylene group may be, for example, a C₆-C₃₀, a C₆-C₂₉, a C₆-C₂₈, a C₆-C₂₇, a C₆-C₂₆, a C₆-C₂₅, a C₆-C₂₄, a C₆-C₂₃, a C₆-C₂₂, a C₆-C₂₁, a C₆-C₂₀, a C₆-C₁₉, a C₆-C₁₈, a C₆-C₁₇, a C₆-C₁₆, a C₆-C₁₅, a C₆-C₁₄, a C₆-C₁₃, a C₆-C₁₂, a C₆-C₁₁, a C₆-C₁₀, a C₆, a C₁₀, a C₁₂, a C₁₃, a C₁₄, a C₁₅, a C₁₆, a C₁₇, or a C₁₈ aryl group or arylene group, specifically, phenyl, biphenyl, naphthyl, terphenyl, phenanthrene, triphenylene, or the like.

When Ar¹ is a heterocyclic group, the heterocyclic group may be, for example, a C₂-C₃₀, a C₂-C₂₉, a C₂-C₂₈, a C₂-C₂₇, a C₂-C₂₆, a C₂-C₂₅, a C₂-C₂₄, a C₂-C₂₃, a C₂-C₂₂, a C₂-C₂₁, a C₂-C₂₀, a C₂-C₁₉, a C₂-C₁₈, a C₂-C₁₇, a C₂-C₁₆, a C₂-C₁₅, a C₂-C₁₄, a C₂-C₁₃, a C₂-C₁₂, a C₂-C₁₁, a C₂-C₁₀, a C₂-C₉, a C₂-C₈, a C₂-C₇, a C₂-C₆, a C₂-C₅, a C₂-C₄, a C₂-C₃, a C₂, a C₃, a C₄, a C₅, a C₆, a C₇, a C₈, a C₉, a C₁₀, a C₁₁, a C₁₂, a C₁₃, a C₁₄, a C₁₅, a C₁₆, a C₁₇, a C₁₈, a C₁₉, a C₂₀, a C₂₁, a C₂₂, a C₂₃, a C₂₄, a C₂₅, a C₂₆, a C₂₇, a C₂₈, or a C₂₉ heterocyclic group, specifically, pyridine, pyrimidine, pyrazine, pyridazine, triazine, furan, pyrrole, indene, indole, phenyl-indole, benzoindole, phenyl-benzoindole, pyrazinoindol, quinoline, isoquinoline, benzoquinoline, pyridoquinoline, quinazoline, benzoquinazoline, dibenzoquinazoline, phenanthroquinazoline, quinoxaline, benzoquinoxaline, dibenzoquinoxaline, benzofuran, naphthobenzofuran, dibenzofuran, dinaphthofuran, thiophene, benzothiophene, dibenzothiophene, naphthobenzothiophene, dinaphthothiophene, carbazole, phenyl-carbazole, benzocarbazole, phenyl-benzocarbazole, naphthyl-benzocarbazole, dibenzocarbazole, indolocarbazole, benzofuropyridine, benzothienopyridine, benzofuropyridine, benzothienopyrimidine, benzofuropyrimidine, benzothienopyrazine, benzofuropyrazine, benzoimidazole, benzothiazole, benzooxazole, benzosiloe, phenanthroline, dihydro-phenylphenazine, 10-phenyl-10H-phenoxazine, phenoxazine, phenothiazine, dibenzodioxin, benzodibenzodioxin, thianthrene, 9,9-dimethyl-9H-xanthene, 9,9-dimethyl-9H-thioxanthene, dihydrodimethylphenylacridine, spiro[fluorene-9,9'-xanthene] and the like.

When at least one of R³ to R⁶ is an alicyclic group, the alicyclic group may be, for example, a C₃-C₃₀, a C₃-C₂₉, a C₃-C₂₈, a C₃-C₂₇, a C₃-C₂₆, a C₃-C₂₅, a C₃-C₂₄, a C₃-C₂₃, a C₃-C₂₂, a C₃-C₂₁, a C₃-C₂₀, a C₃-C₁₉, a C₃-C₁₈, a C₃-C₁₇, a C₃-C₁₆, a C₃-C₁₅, a C₃-C₁₄, a C₃-C₁₃, a C₃-C₁₂, a C₃-C₁₁, a C₃-C₁₀, a C₃-C₈, a C₃-C₆, a C₆, a C₁₀, a C₁₁, a C₁₂, a C₁₃, a C₁₄, a C₁₅, a C₁₆, a C₁₇ or a C₁₈ alicyclic group, specifically, cyclobutane, cyclopentane, cyclohexane, cyclohexene, bicycloheptane, adamantyl, etc.

When Ar^{a} is a fluorenyl group or Ar¹ is a fluorenylene group, the fluorenyl group or fluorenylene group may be, for example, 9,9-dimethyl-9H-fluorene, 9,9-diphenyl-9H-fluorene, 9,9'-spirobifluorene, spiro[benzo[b]fluorene-11,9'-fluorene], benzo[b]fluorene, 11,11-diphenyl-11H-benzo[b]fluorene, 9-(naphthalen-2-yl)-9-phenyl-9H-fluorene, or the like.

When at least one of R¹ to R⁶, R^{a}, R^{b} is an alkyl group, the alkyl group may be, for example, a C₁-C₂₀, a C₁-C₁₀, a C₁-C₄, a C₁, a C₂, a C₃, or a C₄ alkyl group, for example, a methyl group, an ethyl group, a propyl group, an isopropyl group, a butyl group, a tert-butyl group, a pentyl group or the like, and the hydrogen of the alkyl group may be replaced by deuterium.

Ar¹, Ar^{a}, R³ to R⁶, R^{a} to R^{c} may be each substituted with one or more substituents selected from the group consisting of deuterium; halogen; a silane group unsubstituted or substituted with a C₁-C₂₀ alkyl group or a C₆-C₂₀ aryl group; a phosphine oxide substituted or unsubstituted with a C₁-C₂₀ alkyl group or a C₆-C₂₀ aryl group; a cyano group; a nitro group; a C₁-C₂₀ alkylthio group; a C₁-C₂₀ alkoxy group; a C₆-C₃₀ aryloxy group; a C₆-C₃₀ arylthio group; a C₁-C₂₀ alkyl group; a C₂-C₂₀ alkenyl group; a C₂-C₂₀ alkynyl group; a C₆-C₃₀ aryl group; a deuterium-substituted C₆-C₃₀ aryl group; a C₃-C₃₀ alicyclic group; a fused ring of a C₆-C₃₀ aromatic ring and a C₃-C₃₀ alicyclic group; and a C₂-C₃₀ heterocyclic group comprising at least one heteroatom selected from the group consisting of O, N, S, Si and P, and the adjacent substituents may be bonded to each other to form a ring, and hydrogen of the substituents may be replaced with deuterium. With the proviso that the substituents of R^{a} and the substituents of R^{b} do not form a ring.

When at least one of Ar¹, Ar^{a}, R³ to R⁶, and R^{a} to R^{c} is substituted with an aryl group, the aryl group may be, for example, a C₆-C₃₀, C₆-C₂₉, C₆-C₂₈, C₆-C₂₇, C₆-C₂₆, C₆-C₂₅, C₆-C₂₄, C₆-C₂₃, C₆-C₂₂, C₆-C₂₁, C₆-C₂₀, C₆-C₁₉, C₆-C₁₈, C₆-C₁₇, C₆-C₁₆, C₆-C₁₅, C₆-C_{14,} C₆-C₁₃, C₆-C₁₂, C₆-C₁₁, C₆-C₁₀, C₆, C₁₀, C₁₂, C₁₃, C₁₄, C₁₅, C₁₆, C₁₇, or C₁₈ aryl group.

When at least one of Ar¹, Ar^{a}, R³ to R⁶, and R^{a} to R^{c} is substituted with an alicyclic group, the alicyclic group may be, for example, a C₃-C₃₀, C₃-C₂₉, C₃-C₂₈, C₃-C₂₇, C₃-C₂₆, C₃-C₂₅, C₃-C₂₄, C₃-C₂₃, C₃-C₂₂, C₃-C₂₁, C₃-C₂₀, C₃-C₁₉, C₃-C₁₈, C₃-C₁₇, C₃-C₁₆, C₃-C₁₅, C₃-C₁₄, C₃-C₁₃, C₃-C₁₂, C₃-C₁₁, C₃-C₁₀, C₃-C₈, C₃-C₆, C₆, C₁₀, C₁₁, C₁₂, C₁₃, C₁₄, C₁₅, C₁₆, C₁₇, or C₁₈ alicyclic group.

When at least one of Ar¹, Ar^{a}, R³ to R⁶, and R^{a} to R^{c} is substituted with an alkyl group, the alkyl group may be, for example, a C₁-C₂₀, C₁-C₁₀, C₁-C₄, C₁, C₂, C₃, or C₄ alkyl group, for example, a methyl group, an ethyl group, a propyl group, an isopropyl group, a butyl group, a tert-butyl group, or the like.

Formula 1 may be represented by one of Formula 1-1 to Formula 1-4.

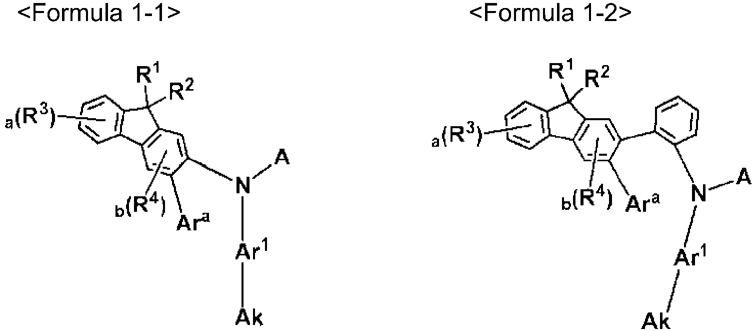

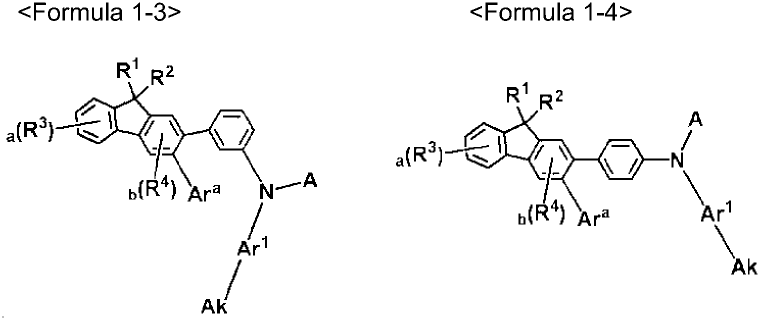

In Formula 1-1 to Formula 1-4, A, R¹ to R⁴, Ar¹, Ar^{a}, Ak, a and b are the same as defined in Formula 1.

Formula A may be represented by one of Formula A-1 to Formula A-4.

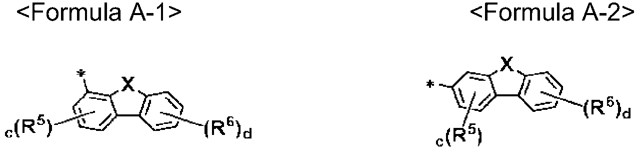

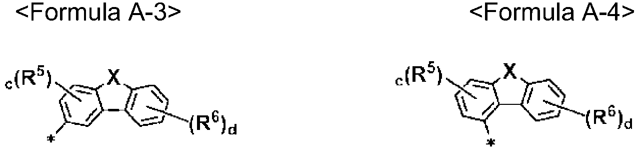

In Formula A-1 to Formula A-4, X, R⁵, R⁶, c and d are the same as defined in Formula 1.

Ar¹ may be selected from the group consisting of Formula L-1 to Formula L-27, and L¹ may be a single bond or selected from the group consisting of Formula L-1 to Formula L-27

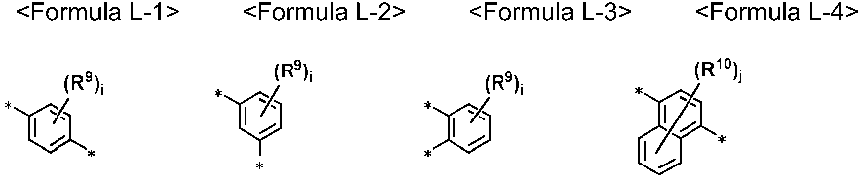

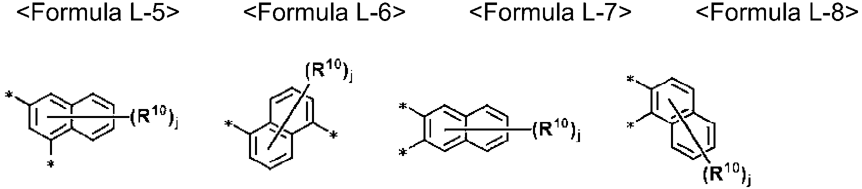

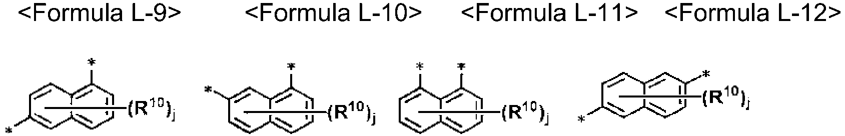

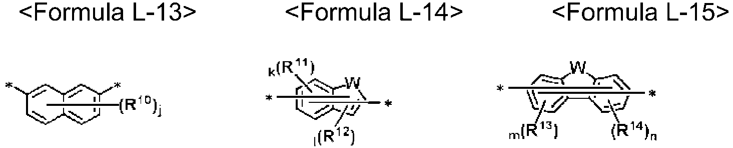

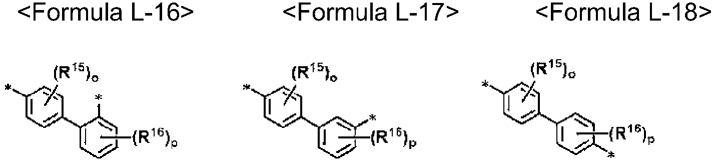

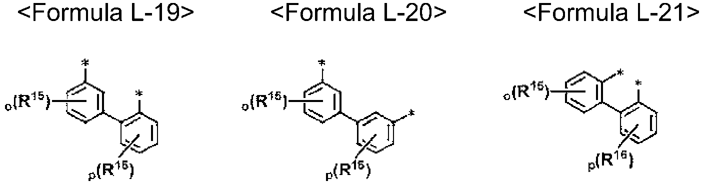

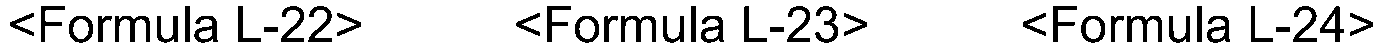

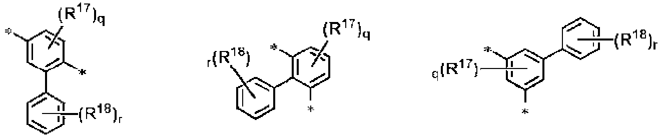

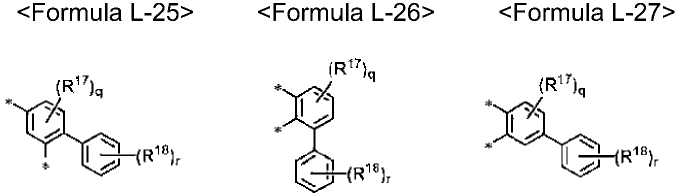

In Formula L-1 to Formula L-27, each symbol is defined as follows.

W is O, S, C(R₁), C(R₂)(R₃), N or N(Ar₁), provided that when L-14 and L-15 are connected via W, W is C(R₁) or N.

R⁹ to R¹⁸, R₁ to R₃ are each independently selected from the group consisting of hydrogen; deuterium; halogen; a silane group unsubstituted or substituted with a C₁-C₂₀ alkyl group or a C₆-C₂₀ aryl group; a phosphine oxide substituted or unsubstituted with a C₁-C₂₀ alkyl group or a C₆-C₂₀ aryl group; a cyano group; a nitro group; a C₁-C₂₀ alkylthio group; a C₁-C₂₀ alkoxy group; a C₆-C₃₀ aryloxy group; a C₆-C₃₀ arylthio group; a C₁-C₂₀ alkyl group; a C₂-C₂₀ alkenyl group; a C₂-C₂₀ alkynyl group; a C₆-C₃₀ aryl group; a deuterium-substituted C₆-C₃₀ aryl group; a C₃-C₃₀ alicyclic group; a fused ring of a C₆-C₃₀ aromatic ring and a C₃-C₃₀ alicyclic group; and a C₂-C₃₀ heterocyclic group comprising at least one heteroatom selected from the group consisting of O, N, S, Si and P, and adjacent groups may be bonded to each other to form a ring, and i, k, m, n, o and p are each an integer of 0 to 4, j is an integer of 0 to 6, I is an integer of 0 to 2, q is an integer of 0 to 3, and r is an integer of 0 to 5.

Ar₁ is selected from the group consisting of a C₁-C₂₀ alkyl group; a C₆-C₃₀ aryl group; a deuterium-substituted C₆-C₃₀ aryl group; a C₃-C₃₀ alicyclic group; a fused ring of a C₆-C₃₀ aromatic ring and a C₃-C₃₀ alicyclic group; and a C₂-C₃₀ heterocyclic group comprising at least one heteroatom selected from the group consisting of O, N, S, Si and P.

Specifically, compound represented by Formula 1 may be one of the following compounds, but is not limited thereto.

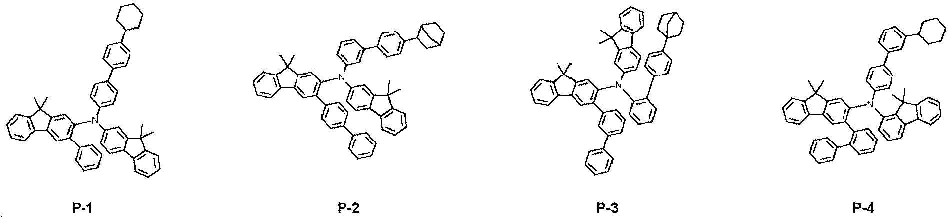

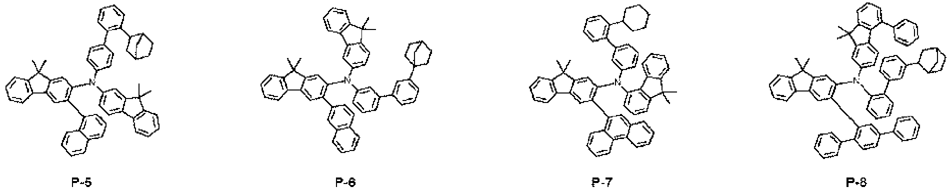

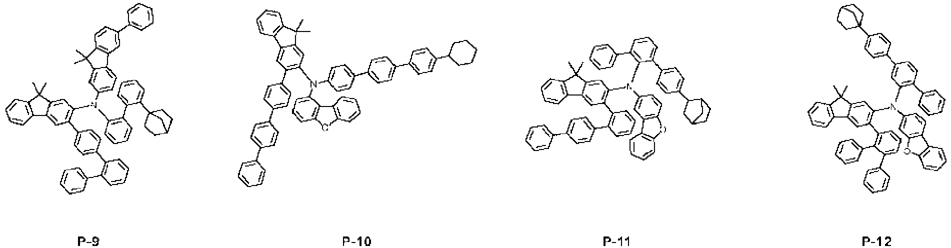

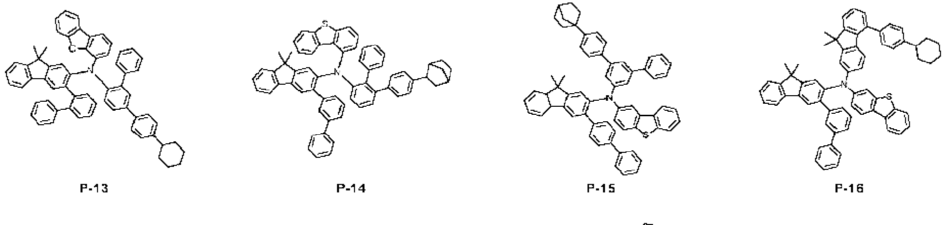

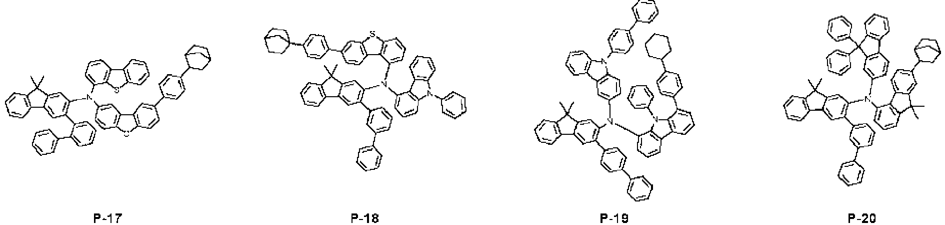

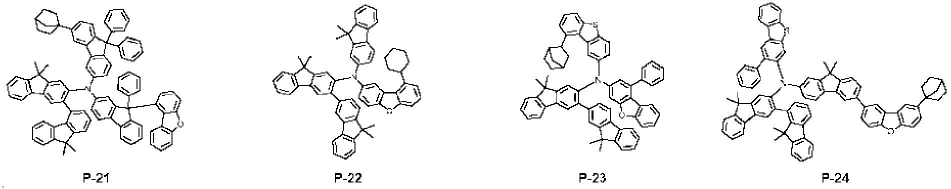

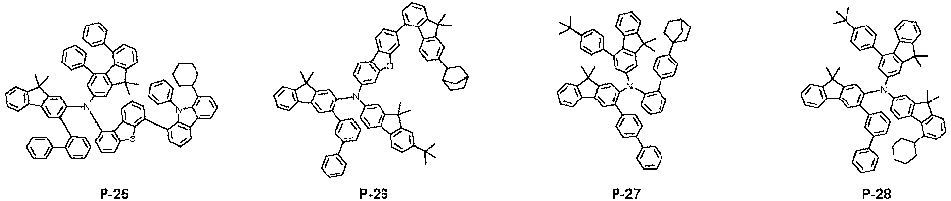

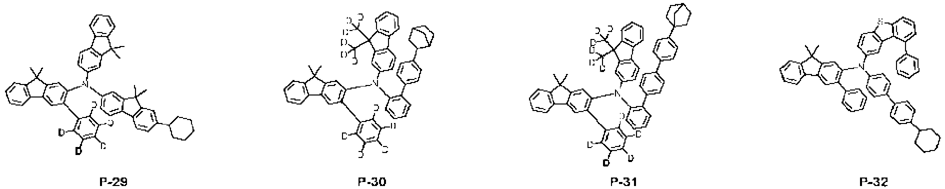

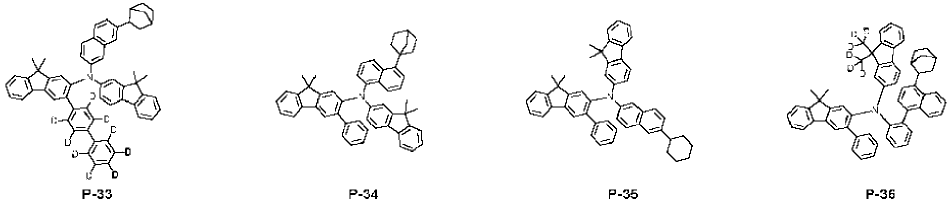

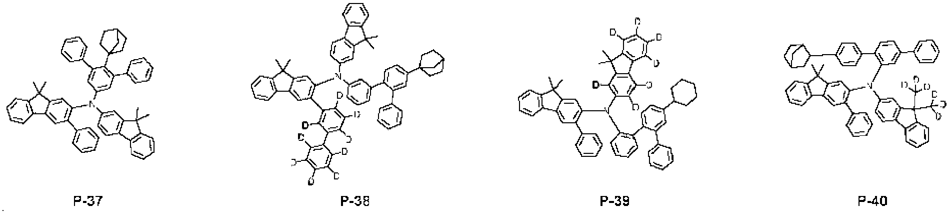

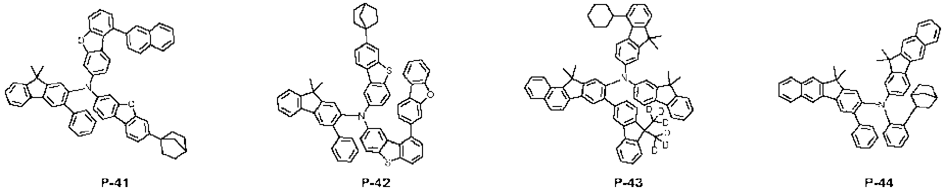

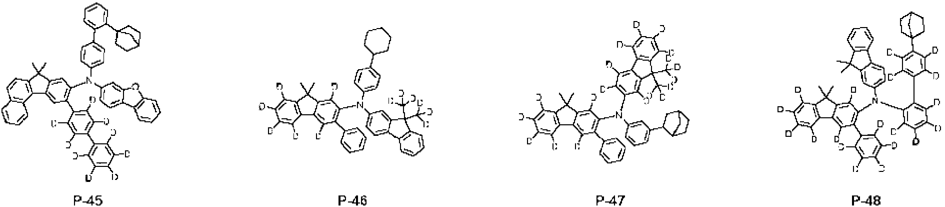

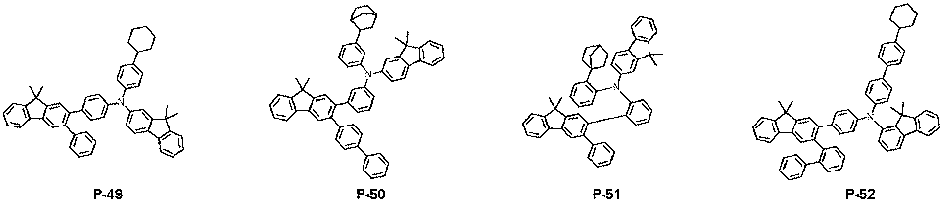

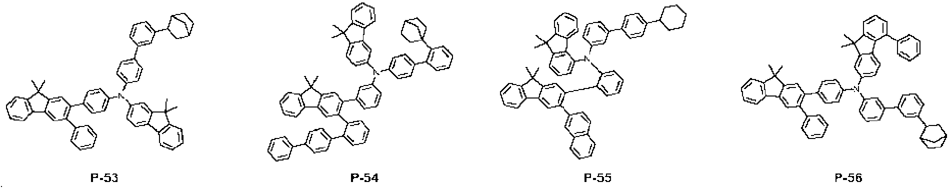

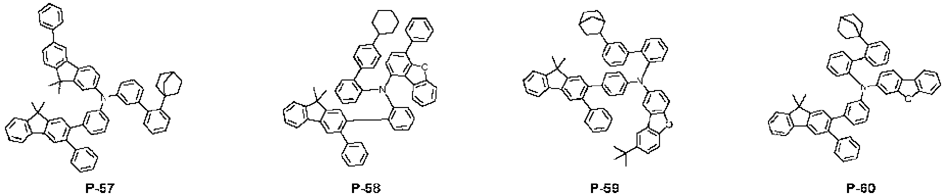

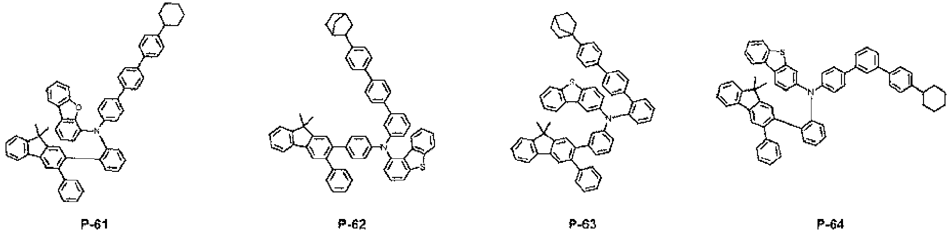

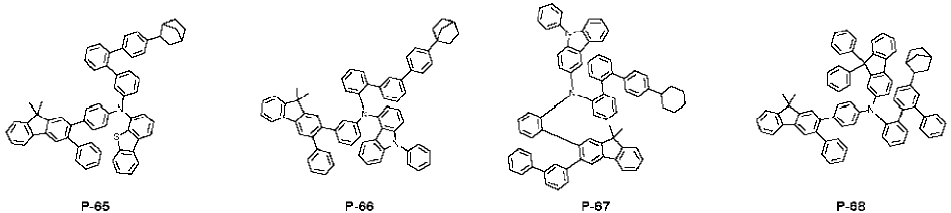

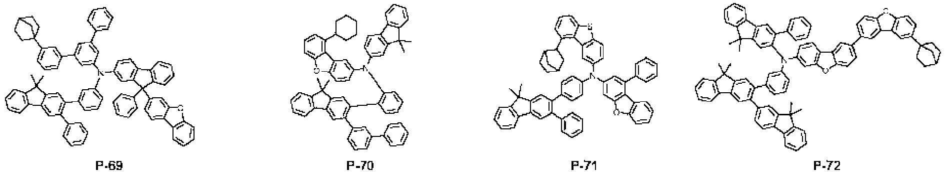

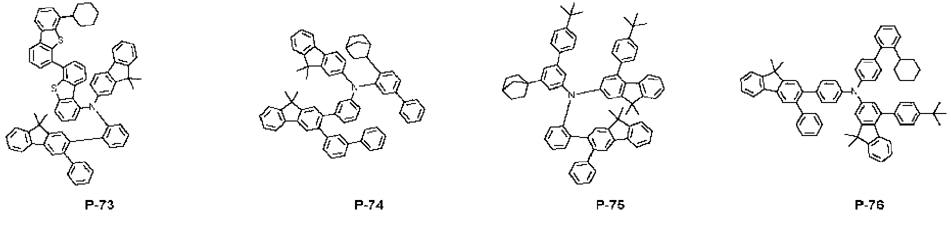

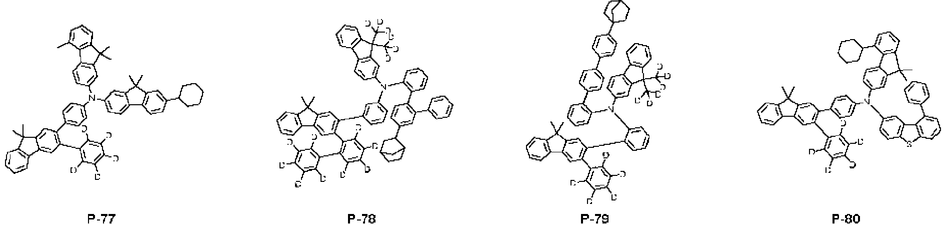

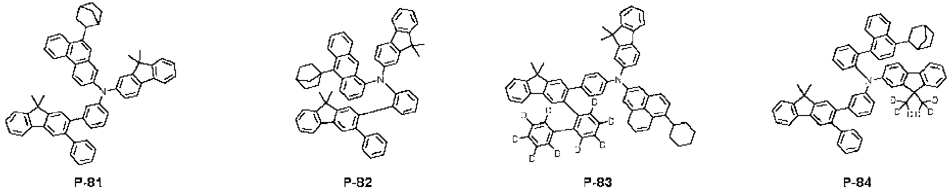

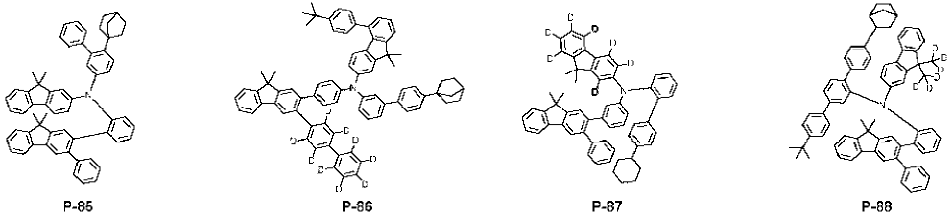

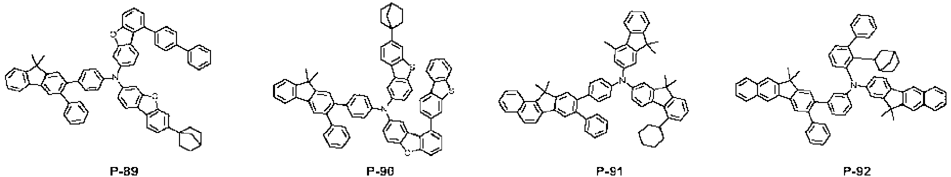

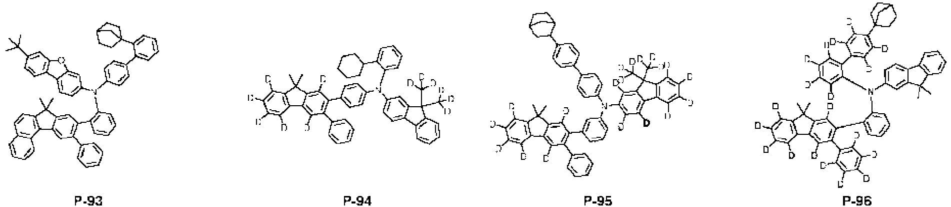

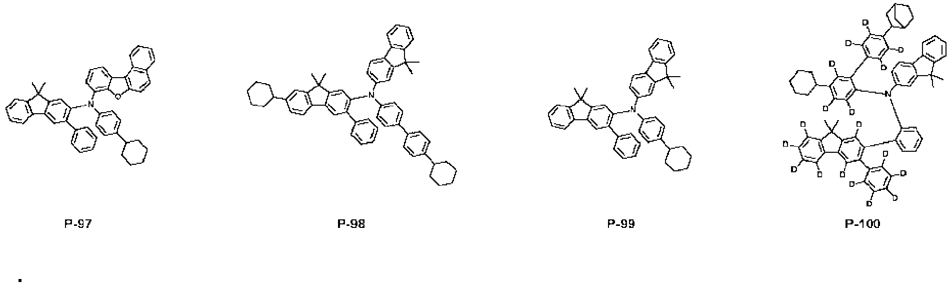

In another aspect, the present invention provides an organic electronic device comprising a first electrode, a second electrode, and an organic layer formed between the first electrode and the second electrode, wherein the organic layer comprises the compound of Formula 1, and preferably the compound is comprised in an emission auxiliary layer.

In another aspect, the present invention provides an electronic apparatus comprising a display device comprising an organic electronic device, and a controller configured to drive the display device, wherein the organic electronic device comprises compound represented by Formula 1.

The reorganization energy of a compound used as material of an emission auxiliary layer may affect the performance of an organic electroluminescent device, and the reorganization energy will be described below.

Reorganization energy is the energy lost due to changes in molecular geometry during charge (electron or hole) transfer. It is dependent on molecular geometry and tends to be smaller when the difference between the potential energy surfaces (PES) of the neutral and charged states is smaller. The RE value can be obtained using the following equation. ${RE}_{hole} : {λ}^{+} = \left({E}_{NOCE}-{E}_{COCE}\right) + \left({E}_{CONE}-{E}_{NONE}\right)$${RE}_{elec} : {λ}^{-} = \left({E}_{NOAE}-{E}_{AOAE}\right) + \left({E}_{AONE}-{E}_{NONE}\right)$
NONE: Neutral geometry of neutral molecular (= NO opt.)
NOAE: Anion geometry of neutral molecular
NOCE: Cation geometry of neutral molecular
AONE: Neutral geometry of Anion molecular
AOAE: Anion geometry of Anion molecular (= AO opt.)
CONE: Neutral geometry of cation molecular
COCE: Cation geometry of cation molecular (= CO opt.)

Reorganization energy and charge mobility have an inverse relationship. When *r* and *T* are held constant, the RE value directly influences the mobility of each material.

The relationship between reorganization energy (RE) and mobility can be expressed as the following equation, and it is described based on the charge transfer matrix element.$μ = k \frac{{r}^{2}}{2 {k}_{B} T / e}$ $k = \left(\frac{4 {π}^{2}}{h}\right) \frac{{t}^{2}}{\sqrt{4 {πλk}_{B} T}} exp \left[-\frac{λ}{4 {k}_{B} T}\right]$
(*λ*: Reorganization energy, *µ*: mobility, r: dimer displacement, t: intermolecular charge transfer matrix element)

As shown in the equation above, charge mobility increases as the RE value decreases.

To calculate the reorganization energy, a simulation tool that can compute the potential energy of a molecule based on its geometry is necessary. For example, Gaussian09 (hereinafter referred to as G09) and the Jaguar (hereinafter referred to as JG) module in Schrödinger Materials Science can be used. Both G09 and JG are quantum mechanical (QM) computational tools used for analyzing molecular properties, and they are capable of optimizing molecular structures and computing the single-point energy for a given molecular structure.

Quantum mechanical (QM) calculations performed on molecular structures require substantial computational resources. For example, such calculations may be executed using two cluster servers, each comprising four node workstations and one master workstation. Each node workstation is capable of performing molecular QM calculations via parallel processing based on symmetric multiprocessing (SMP), utilizing CPUs having 36 or more cores.

Using Gaussian 09 (G09), molecular structures optimized for neutral and charged states are calculated along with their corresponding potential energies (NONE and COCE), which are necessary for determining the reorganization energy. Subsequently, by altering only the charge of each of the two optimized structures, the potential energy of the neutral-state-optimized structure under the charged state (NOCE) and the potential energy of the charged-state-optimized structure under the neutral state (CONE) are calculated. The reorganization energy is then determined based on the following relationship.${RE}_{charge} : λ = \left({E}_{NOCE}-{E}_{COCE}\right) + \left({E}_{CONE}-{E}_{NONE}\right)$

Since Schrödinger provides a function for automatically performing the aforementioned calculation process, the JG module is capable of sequentially calculating the potential energies of each state and determining the rearrangement energy (RE) value by simply inputting the molecular structure (NO) of the ground state.

In another aspect, the present invention provides a compound represented by Formula 1, obtained by recovering a material of the organic layer from a deposition apparatus after depositing the organic layer in a manufacturing process of an organic electronic device, followed by purification. The compound obtained by recovery and purification has a purity of 99.9% or higher.

In another aspect, the present invention provides a method for recovering a compound represented by Formula 1, the method comprising: depositing an organic layer material comprising the compound represented by Formula 1; recovering the organic layer material that is attached to a deposition apparatus; and purifying the recovered organic layer material to obtain the compound represented by Formula 1 having a purity of 99.9% or higher.

The purification step may comprise: a recrystallization step in which the recovered material of an organic layer is recrystallized using a recrystallization solvent; an adsorption and separation step using an adsorbent; and a sublimation and purification step.

The recrystallization step may comprise a preliminary purification process in which a compound represented by Formula 1 having a purity of 98% is obtained using a recrystallization solvent.

As the recrystallization solvent, a polar solvent having a polarity index (PI) of 5.5 to 7.2 is preferably used, or a mixed solvent comprising a polar solvent having a polarity index of 5.5 to 7.2 and a non-polar solvent having a polarity index of 2.0 to 4.7 may be used.

When a mixed solvent of a polar solvent and a non-polar solvent is used as the recrystallization solvent, the non-polar solvent may be used in an amount of 15% (v/v) or less relative to the volume of the polar solvent.

As the recrystallization solvent, a single solvent of methylpyrrolidone (N-methylpyrrolidone; NMP) is preferably used. Alternatively, a mixed polar solvent in which methylpyrrolidone is mixed with at least one solvent selected from the group consisting of 1,3-dimethyl-2-imidazolidinone, 2-pyrrolidone, N,N-dimethylformamide, dimethylacetamide, and dimethyl sulfoxide may be used. In addition, a single or mixed non-polar solvent selected from the group consisting of toluene, dichloromethane (DCM), dichloroethane (DCE), tetrahydrofuran (THF), chloroform, ethyl acetate, and butanone, or a mixture of the polar solvent and the non-polar solvent may also be used.

The preliminary purification process may comprise dissolving the unpurified organic electroluminescent material, which has been recovered from a deposition apparatus, in a polar solvent at a temperature of 90°C to 120°C, and cooling the solution to a temperature of 0°C to 5°C to precipitate crystals.

The preliminary purification process may comprise dissolving the unpurified organic electroluminescent material, recovered from a deposition apparatus, in a polar solvent at a temperature of 90°C to 120°C; cooling the solution to a temperature of 35°C to 40°C; adding a non-polar solvent to the cooled solution; and further cooling the resulting mixture to a temperature of 0°C to 5°C to precipitate crystals.

The preliminary purification process may comprise dissolving the unpurified organic electroluminescent material, recovered from a deposition apparatus, in a non-polar solvent, and concentrating the solution to remove the non-polar solvent while precipitating crystals.

The preliminary purification process may comprise a step of recrystallization using a polar solvent, followed by a subsequent recrystallization step using a non-polar solvent.

In the adsorption separation step using an adsorbent, the adsorbent may be selected from the group consisting of activated carbon, silica gel, alumina, and other materials known for use in adsorption.

Hereinafter, the present invention will be described in further detail with reference to specific examples regarding the synthesis of compounds represented by Formula 1 and the fabrication of an organic electronic device. However, the present invention is not limited to the following examples.

### [Synthesis example 1]

The compound represented by Formula 1 (Final Product) according to the present invention may be prepared as shown in Reaction Scheme 1 below, but is not limited thereto.

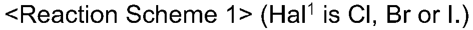

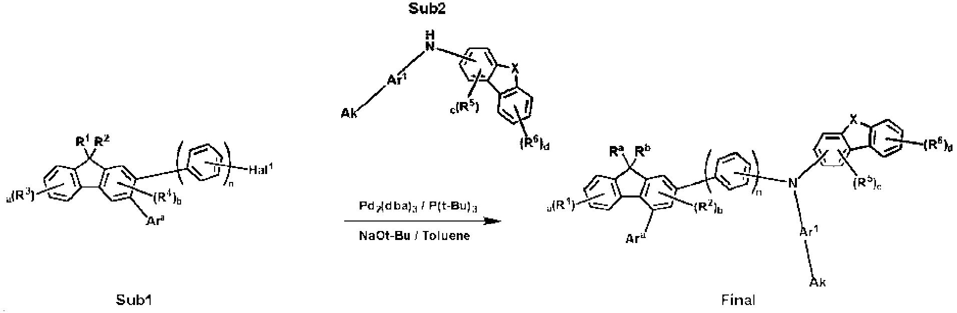

### I. Synthesis Example of Sub1

Sub1 of Reaction Scheme 1 may be synthesized according to Reaction Scheme 2 below, but is not limited thereto.

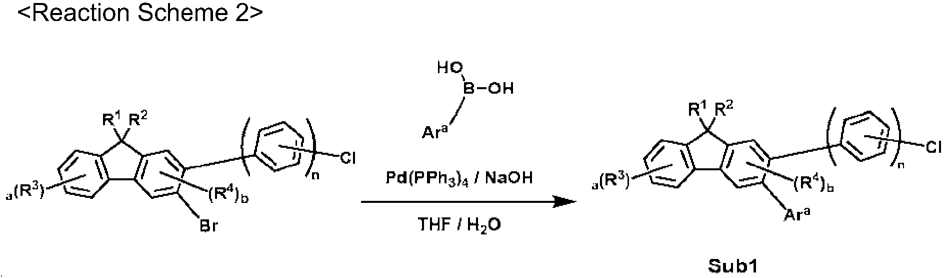

The compounds belonging to Sub 1 may include the following compounds, but are not limited thereto, and the FD-MS (Field Desorption-Mass Spectrometry) values of the following compounds are shown in Table 1.

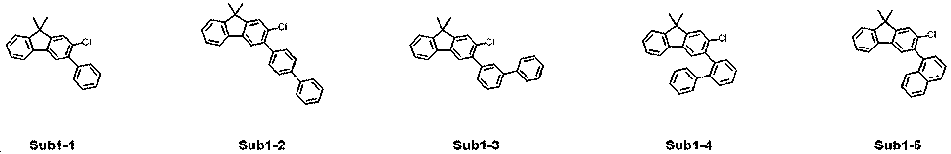

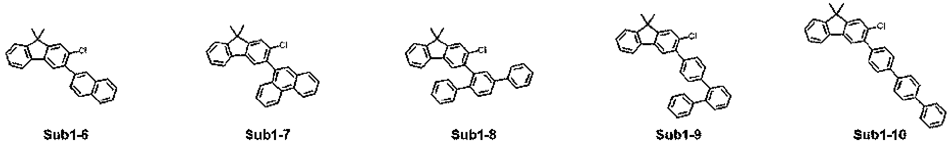

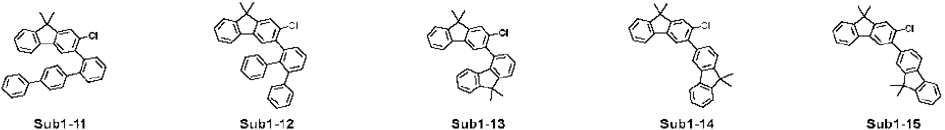

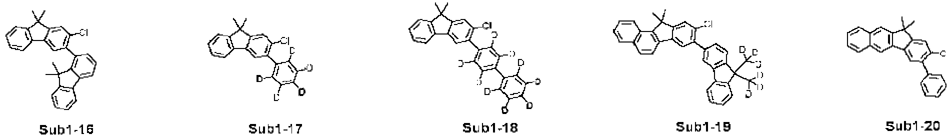

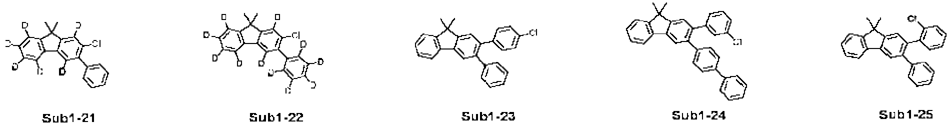

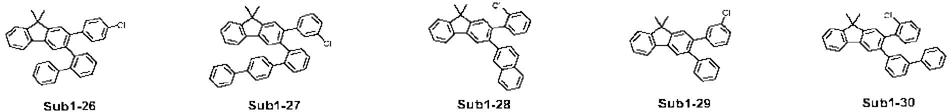

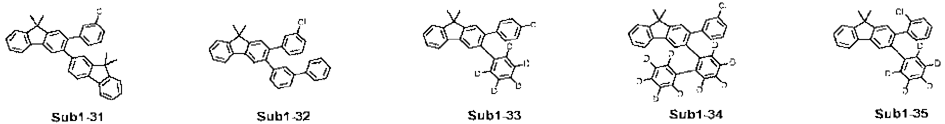

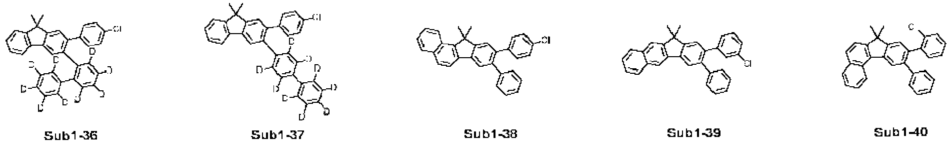

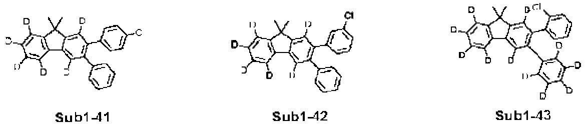

**[Table 1]**

| Compound | FD-MS | Compound | FD-MS |
|---|---|---|---|
| Sub 1-1 | m/z=304.1(C₂₁H₁₇Cl=304.82) | Sub 1-2 | m/z=380.13(C₂₇H₂₁Cl=380.92) |
| Sub 1-3 | m/z=380.13(C₂₇H₂₁Cl=380.92) | Sub 1-4 | m/z=380.13(C₂₇H₂₁Cl=380.92) |
| Sub 1-5 | m/z=354.12(C₂₅H₁₉Cl=354.88) | Sub 1-6 | m/z=354.12(C₂₅H₁₉Cl=354.88) |
| Sub 1-7 | m/z=404.13(C₂₉H₂₁Cl=404.94) | Sub 1-8 | m/z=456.16(C₃₃H₂₅Cl=457.01) |
| Sub 1-9 | m/z=456.16(C₃₃H₂₅Cl=457.01) | Sub 1-10 | m/z=456.16(C₃₃H₂₅Cl=457.01) |
| Sub 1-11 | m/z=456.16(C₃₃H₂₅Cl=457.01) | Sub 1-12 | m/z=456.16(C₃₃H₂₅Cl=457.01) |
| Sub 1-13 | m/z=420.16(C₃₀H₂₅Cl=420.98) | Sub 1-14 | m/z=420.16(C₃₀H₂₅Cl=420.98) |
| Sub 1-15 | m/z=420.16(C₃₀H₂₅Cl=420.98) | Sub 1-16 | m/z=420.16(C₃₀H₂₅Cl=420.98) |
| Sub 1-17 | m/z=309.13(C₂₁H₁₂D₅Cl=309.85) | Sub 1-18 | m/z=389.19(C₂₇H₁₂D₉Cl=389.97) |
| Sub 1-19 | m/z=476.22(C₃₄H₂₁D₆Cl=477.08) | Sub 1-20 | m/z=354.12(C₂₅H₁₉Cl=354.88) |
| Sub 1-21 | m/z=310.14(C₂₁H₁₁D₆Cl=310.85) | Sub 1-22 | m/z=315.17(C₂₁H₆D₁₁Cl=315.88) |
| Sub 1-23 | m/z=380.13(C₂₇H₂₁Cl=380.92) | Sub 1-24 | m/z=456.16(C₃₃H₂₅Cl=457.01) |
| Sub 1-25 | m/z=380.13(C₂₇H₂₁Cl=380.92) | Sub 1-26 | m/z=456.16(C₃₃H₂₅Cl=457.01) |
| Sub 1-27 | m/z=532.2(C₃₉H₂₉Cl=533.11) | Sub 1-28 | m/z=430.15(C₃₁H₂₃Cl=430.98) |
| Sub 1-29 | m/z=380.13(C₂₇H₂₁Cl=380.92) | Sub 1-30 | m/z=456.16(C₃₃H₂₅Cl=457.01) |
| Sub 1-31 | m/z=496.2(C₃₆H₂₉Cl=497.08) | Sub 1-32 | m/z=456.16(C₃₃H₂₅Cl=457.01) |
| Sub 1-33 | m/z=385.16(C₂₇H₁₆D₅Cl=385.95) | Sub 1-34 | m/z=465.22(C₃₃H₁₆D₉Cl=466.07) |
| Sub 1-35 | m/z=385.16(C₂₇H₁₆D₅Cl=385.95) | Sub 1-36 | m/z=465.22(C₃₃H₁₆D₉Cl=466.07) |
| Sub 1-37 | m/z=465.22(C₃₃H₁₆D₉Cl=466.07) | Sub 1-38 | m/z=430.15(C₃₁H₂₃Cl=430.98) |
| Sub 1-39 | m/z=430.15(C₃₁H₂₃Cl=430.98) | Sub 1-40 | m/z=430.15(C₃₁H₂₃Cl=430.98) |
| Sub 1-41 | m/z=386.17(C₂₇H₁₅D₆Cl=386.95) | Sub 1-42 | m/z=386.17(C₂₇H₁₅D₆Cl=386.95) |
| Sub 1-43 | m/z=391.2(C₂₇H₁₀D₁₁Cl=391.98) | | |

### II. Synthesis Example of Sub2

Sub2 of Reaction Scheme 1 may be synthesized according to Reaction Scheme 3 below, but is not limited thereto.

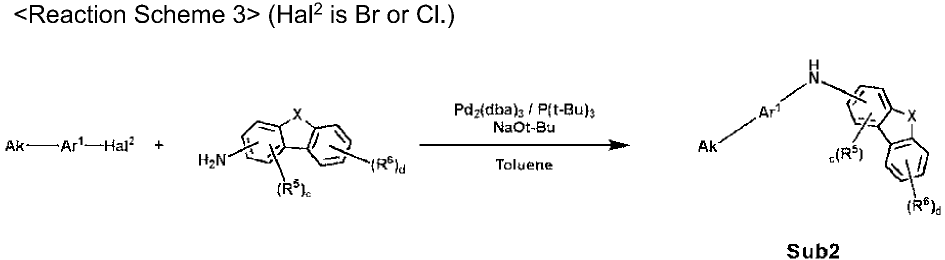

The compounds belonging to Sub2 may include the following compounds, but are not limited thereto, and the FD-MS values of the following compounds are shown in Table 2.

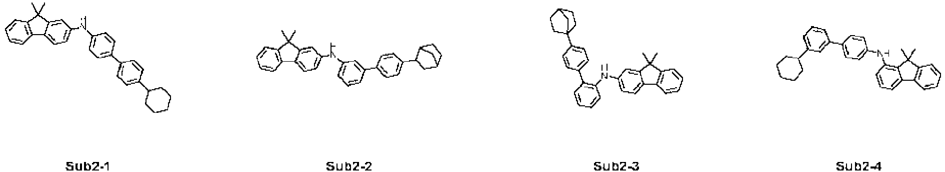

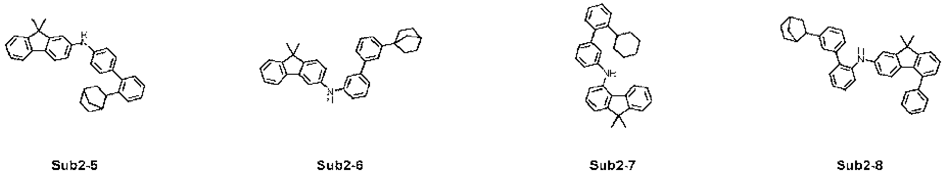

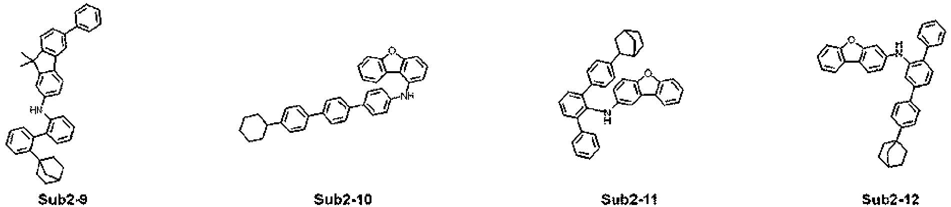

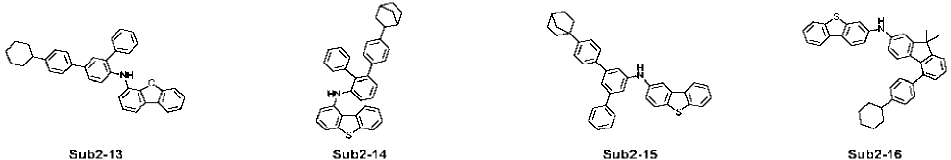

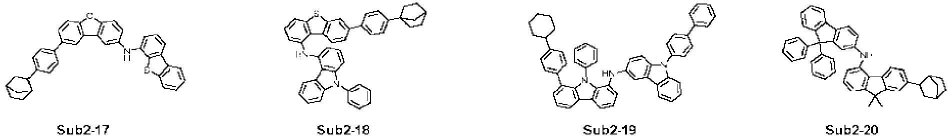

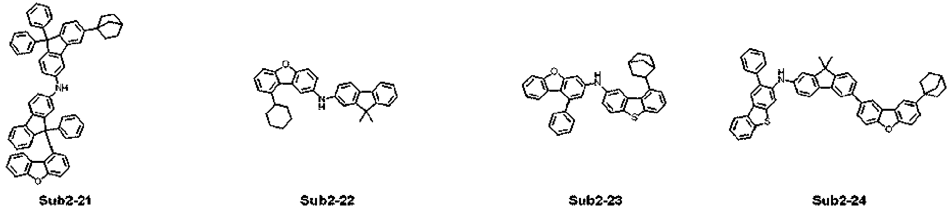

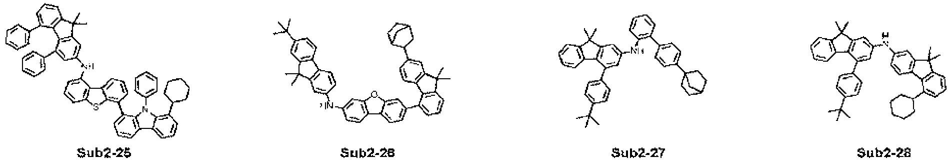

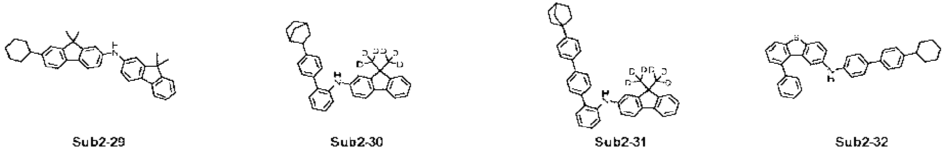

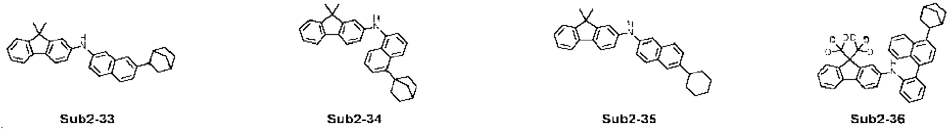

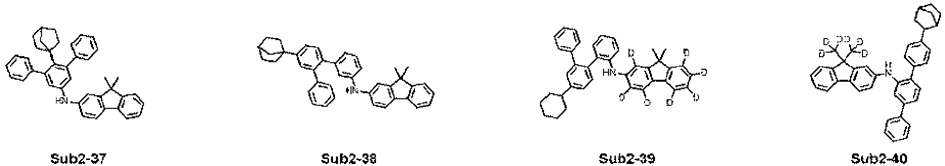

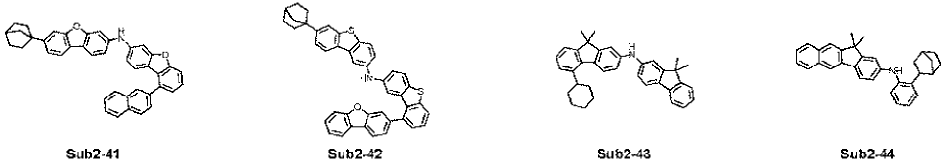

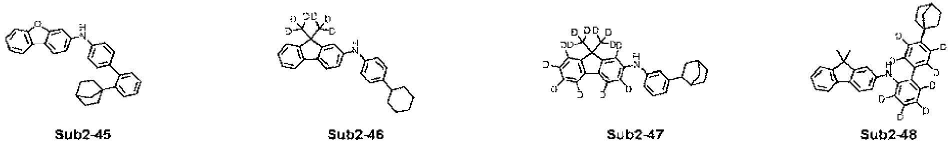

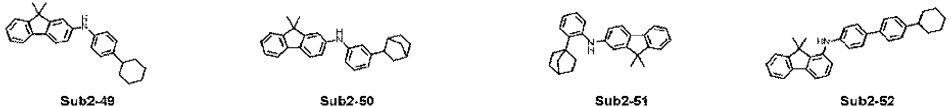

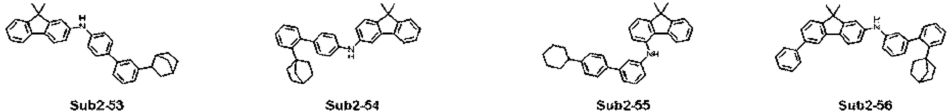

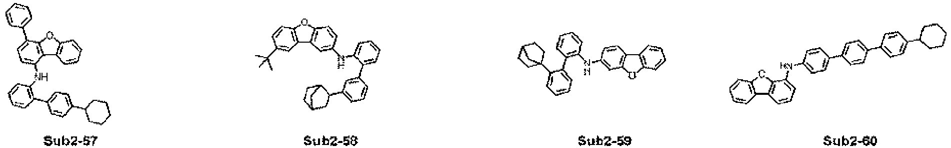

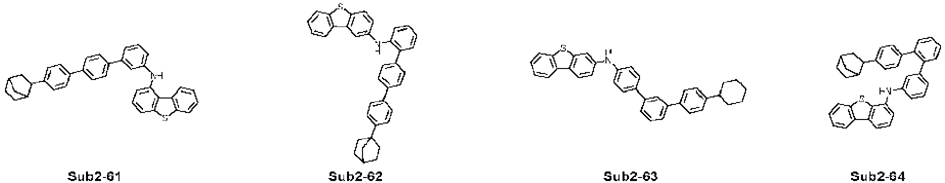

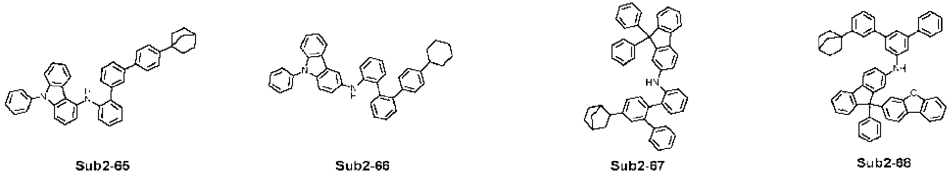

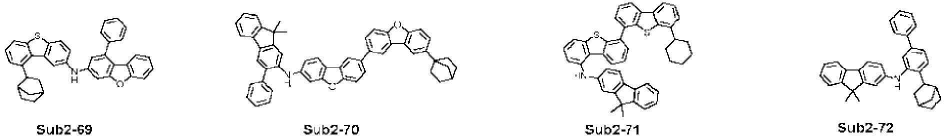

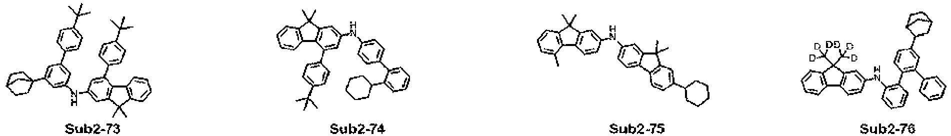

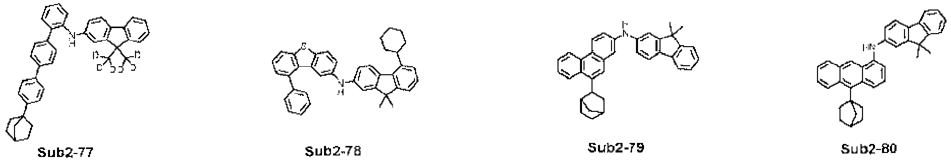

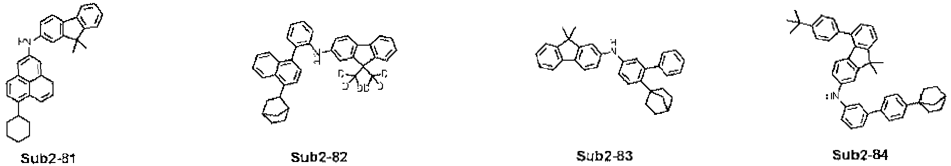

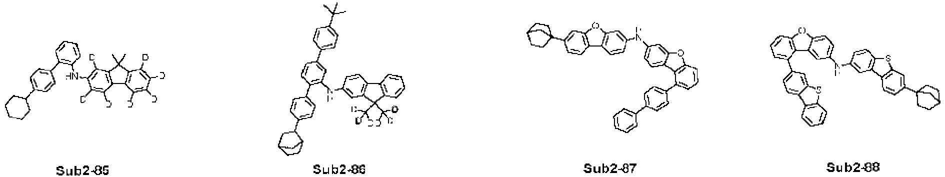

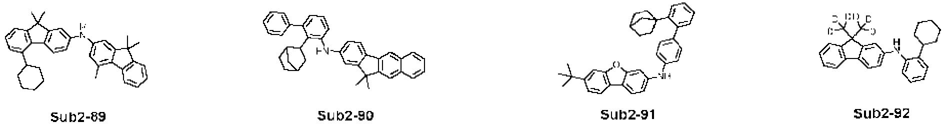

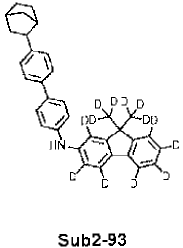

**[Table 2]**

| Compound | FD-MS | Compound | FD-MS |
|---|---|---|---|
| Sub 2-1 | m/z=443.26(C₃₃H₃₃N=443.63) | Sub 2-2 | m/z=455.26(C₃₄H₃₃N=455.65) |
| Sub 2-3 | m/z=455.26(C₃₄H₃₃N=455.65) | Sub 2-4 | m/z=443.26(C₃₃H₃₃N=443.63) |
| Sub 2-5 | m/z=455.26(C₃₄H₃₃N=455.65) | Sub 2-6 | m/z=455.26(C₃₄H₃₃N=455.65) |
| Sub 2-7 | m/z=443.26(C₃₃H₃₃N=443.63) | Sub 2-8 | m/z=531.29(C₄₀H₃₇N=531.74) |
| Sub 2-9 | m/z=531.29(C₄₀H₃₇N=531.74) | Sub 2-10 | m/z=493.24(C₃₆H₃₁NO=493.65) |
| Sub 2-11 | m/z=505.24(C₃₇H₃₁NO=505.66) | Sub 2-12 | m/z=505.24(C₃₇H₃₁NO=505.66) |
| Sub 2-13 | m/z=493.24(C₃₆H₃₁NO=493.65) | Sub 2-14 | m/z=521.22(C₃₇H₃₁NS=521.72) |
| Sub 2-15 | m/z=521.22(C₃₇H₃₁NS=521.72) | Sub 2-16 | m/z=549.25(C₃₉H₃₅NS=549.78) |
| Sub 2-17 | m/z=535.2(C₃₇H₂₉NOS=535.71) | Sub 2-18 | m/z=610.24(C₄₃H₃₄N₂S=610.82) |
| Sub 2-19 | Mass:733.35C54H43N3733.96) | Sub 2-20 | m/z=619.32(C₄₇H₄₁N=619.85) |
| Sub 2-21 | m/z=833.37(C₆₃H₄₇NO=834.08) | Sub 2-22 | m/z=457.24(C₃₃H₃₁NO=457.62) |
| Sub 2-23 | m/z=535.2(C₃₇H₂₉NOS=535.71) | Sub 2-24 | m/z=727.29(C₅₂H₄₁NOS=727.97) |
| Sub 2-25 | m/z=866.37(C₆₃H₅₀N₂S=867.17) | Sub 2-26 | m/z=717.4(C₅₃H₅₁NO=718) |
| Sub 2-27 | m/z=587.36(C₁₄H₄₅N=587.85) | Sub 2-28 | m/z=615.39(C₄₆H₄₉N=615.91) |
| Sub 2-29 | m/z=483.29(C₃₆H₃₇N=483.7) | Sub 2-30 | m/z=461.3(C₃₄H₂₇D₆N=461.68) |
| Sub 2-31 | m/z=537.33(C₄₀H₃₁D₆N=537.78) | Sub 2-32 | m/z=509.22(C₃₆H₃₁NS=509.71) |
| Sub 2-33 | m/z=429.25(C₃₂H₃₁N=429.61) | Sub 2-34 | m/z=429.25(C₃₂H₃₁N=429.61) |
| Sub 2-35 | m/z=417.25(C₃₁H₃₁N=417.6) | Sub 2-36 | m/z=511.31(C₃₈H₂₉D₆N=511.74) |
| Sub 2-37 | m/z=531.29(C₄₀H₃₇N=531.74) | Sub 2-38 | m/z=531.29(C₄₀H₃₇N=531.74) |
| Sub 2-39 | m/z=526.34(C₃₉H₃₀D₇N=526.77) | Sub 2-40 | m/z=537.33(C₄₀H₃₁D₆N=537.78) |
| Sub 2-41 | m/z=569.24(C₄₁H₃₁NO₂=569.7) | Sub 2-42 | m/z=641.18(C₄₃H₃₁NOS₂=641.85) |
| Sub 2-43 | m/z=483.29(C₃₆H₃₇N=483.7) | Sub 2-44 | m/z=429.25(C₃₂H₃₁N=429.61) |
| Sub 2-45 | m/z=429.21(C₃₁H₂₇NO=429.56) | Sub 2-46 | m/z=373.27(C₂₇H₂₃D₆N=373.57) |
| Sub 2-47 | m/z=392.31(C₂₈H₁₆D₁₃N=392.63) | Sub 2-48 | m/z=463.31(C₃₄H₂₅D₈N=463.69) |
| Sub 2-49 | m/z=367.23(C₂₇H₂₉N=367.54) | Sub 2-50 | m/z=379.23(C₂₈H₂₉N=379.55) |
| Sub 2-51 | m/z=379.23(C₂₈H₂₉N=379.55) | Sub 2-52 | m/z=443.26(C₃₃H₃₃N=443.63) |
| Sub 2-53 | m/z=455.26(C₃₄H₃₃N=455.65) | Sub 2-54 | m/z=455.26(C₃₄H₃₃N=455.65) |
| Sub 2-55 | m/z=443.26(C₃₃H₃₃N=443.63) | Sub 2-56 | m/z=531.29(C₄₀H₃₇N=531.74) |
| Sub 2-57 | m/z=493.24(C₃₆H₃₁NO=493.65) | Sub 2-58 | m/z=485.27(C₃₅H₃₅NO=485.67) |
| Sub 2-59 | m/z=429.21(C₃₁ H₂₇NO=429.56) | Sub 2-60 | m/z=493.24(C₃₆H₃₁NO=493.65) |
| Sub 2-61 | m/z=521.22(C₃₇H₃₁NS=521.72) | Sub 2-62 | m/z=521.22(C₃₇H₃₁NS=521.72) |
| Sub 2-63 | m/z=509.22(C₃₆H₃₁NS=509.71) | Sub 2-64 | m/z=521.22(C₃₇H₃₁NS=521.72) |
| Sub 2-65 | m/z=580.29(C₄₃H₃₆N₂=580.78) | Sub 2-66 | m/z=568.29(C₄₂H₃₆N₂=568.76) |
| Sub 2-67 | m/z=655.32(C₅₀H₄₁N=655.88) | Sub 2-68 | m/z=745.33(C₅₆H₄₃NO=745.97) |
| Sub 2-69 | m/z=535.2(C₃₇H₂₉NOS=535.71) | Sub 2-70 | m/z=711.31(C₅₂H₄₁NO₂=711.91) |
| Sub 2-71 | m/z=655.24(C₄₅H₃₇NS₂=655.92) | Sub 2-72 | m/z=455.26(C₃₄H₃₃N=455.65) |
| Sub 2-73 | m/z=643.42(C₄₈H₅₃N=643.96) | Sub 2-74 | m/z=575.36(C₄₃H₄₅N=575.84) |
| Sub 2-75 | m/z=497.31(C₃₇H₃₉N=497.73) | Sub 2-76 | m/z=537.33(C₄₀H₃₁D₆N=537.78) |
| Sub 2-77 | m/z=537.33(C₄₀H₃₁D₆N=537.78) | Sub 2-78 | m/z=549.25(C₃₉H₃₅NS=549.78) |
| Sub 2-79 | m/z=479.26(C₃₆H₃₃N=479.67) | Sub 2-80 | m/z=479.26(C₃₆H₃₃N=479.67) |
| Sub 2-81 | m/z=455.26(C₃₄H₃₃N=455.65) | Sub 2-82 | m/z=511.31(C₃₈H₂₉D₆N=511.74) |
| Sub 2-83 | m/z=455.26(C₃₄H₃₃N=455.65) | Sub 2-84 | m/z=587.36(C₄₄H₄₅N=587.85) |
| Sub 2-85 | m/z=450.31(C₃₃H₂₆D₇N=450.68) | Sub 2-86 | m/z=593.39(C₄₄H₃₉D₆N=593.89) |
| Sub 2-87 | m/z=595.25(C₄₃H₃₃NO₂=595.74) | Sub 2-88 | m/z=641.18(C₄₃H₃₁NOS₂=641.85) |
| Sub 2-89 | m/z=497.31(C₃₇H₃₉N=497.73) | Sub 2-90 | m/z=505.28(C₃₈H₃₅N=505.71) |
| Sub 2-91 | m/z=485.27(C₃₅H₃₅NO=485.67) | Sub 2-92 | m/z=373.27(C₂₇H₂₃D₆N=373.57) |
| Sub 2-93 | m/z=468.34(C₃₄H₂₀D₁₃N=468.72) | | |

### III. Synthesis Examples of Final Compounds

### 1. Synthesis Example of P-1

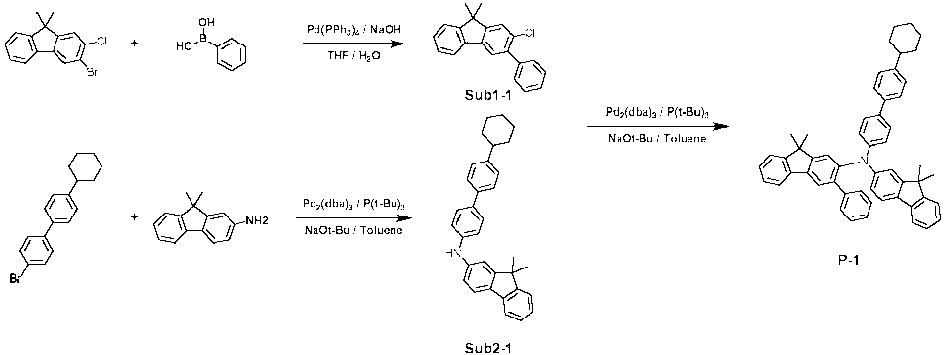

### 1) Synthesis of Sub1-1

3-bromo-2-chloro-9,9-dimethyl-9H-fluorene (50.0 g, 162.5 mmol) was dissolved in THF (542 mL), followed by the addition of phenylboronic acid (21.8 g, 178.8 mmol), NaOH (19.5 g, 487.6 mmol), Pd(PPh₃)₄ (9.39 g, 8.13 mmol), and water (181 mL), and the reaction mixture was stirred under reflux at 80°C. Upon completion of the reaction, extraction was performed using CH₂Cl₂ and water, and the organic layer was dried over MgSO₄ and concentrated. Thereafter, the concentrate was separated by silica gel column and recrystallized to obtain 39.6 g of the product (yield: 80 %).

### 2) Synthesis of Sub2-1

4-bromo-4'-cyclohexyl-1,1'-biphenyl (50.0 g, 158.6 mmol) was dissolved in toluene (529 mL), followed by the addition of 9,9-dimethyl-9H-fluoren-2-amine (39.8 g, 209.29 mmol), Pd₂(dba)₃ (4.36 g, 4.76 mmol), P(t-Bu)₃ (1.93 g, 9.52 mmol), and NaOt-Bu (30.5 g, 317.2 mmol), and the reaction mixture was stirred at 60°C. Upon completion of the reaction, extraction was performed using CH₂Cl₂ and water, and the organic layer was dried over MgSO₄ and concentrated. Thereafter, the concentrate was separated by silica gel column and recrystallized to obtain 58.4 g of the product (yield: 83 %).

### 3) Synthesis of P-1

Sub1-1 (30.0 g, 98.4 mmol) was dissolved in toluene (492 mL), followed by the addition of Sub2-1 (43.7 g, 98.4 mmol), Pd₂(dba)₃ (2.70 g, 2.95 mmol), P(t-Bu)₃ (1.19 g, 5.91 mmol), and NaOt-Bu (28.4 g, 295.3 mmol), and the reaction mixture was stirred under reflux at 125°C. Upon completion of the reaction, extraction was performed using CH₂Cl₂ and water, and the organic layer was dried over MgSO₄ and concentrated. Thereafter, the concentrate was separated by silica gel column and recrystallized to obtain 54 g of the product (yield: 77 %).

### 2. Synthesis Example of P-11

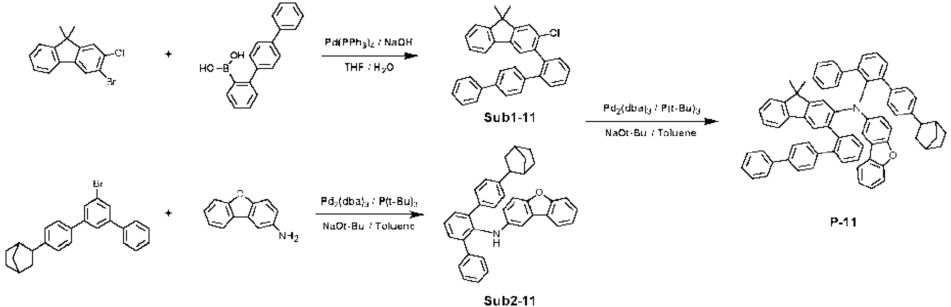

### 1) Synthesis of Sub1-11

3-bromo-2-chloro-9,9-dimethyl-9H-fluorene (50.0 g, 162.5 mmol) was dissolved in THF (542 mL), and then [1,1':4',1"-terphenyl]-2-ylboronic acid (49 g, 178.8 mmol), NaOH (19.5 g, 487.6 mmol), Pd(PPh₃)₄ (9.39 g, 8.13 mmol), and water (181 ml) were added thereto. The reaction was carried out in the same manner as in the preparation of Sub1-1 to obtain 60.2 g of the product (yield: 81%).

### 2) Synthesis of Sub2-11

2-(5'-bromo-[1,1':3',1"-terphenyl]-4-yl)bicyclo[2.2.1]heptane (50.0 g, 124.0 mmol) was dissolved in toluene (529 mL), and then dibenzo[b,d]furan-2-amine (27.3 g, 148.8 mmol), Pd₂(dba)₃ (3.41 g, 3.72 mmol), P(t-Bu)₃ (1.50 g, 7.44 mmol), and NaOt-Bu (23.8 g, 247.9 mmol) were added thereto. The reaction was carried out in the same manner as in the preparation of Sub2-1 to obtain 47.6 g of the product (yield: 76%).

### 3) Synthesis of P-11

**[** Sub1-11 (30.0 g, 65.6 mmol) was dissolved in toluene (328 mL), and then Sub2-11 (33.2 g, 65.6 mmol), Pd₂(dba)₃ (1.80 g, 1.97 mmol), P(t-Bu)₃ (0.80 g, 3.94 mmol), and NaOt-Bu (18.9 g, 196.9 mmol) were added thereto. The reaction was carried out in the same manner as in the preparation of P-1 to obtain 45.6 g of the product (yield: 75%).

### 3. Synthesis Example of P-16

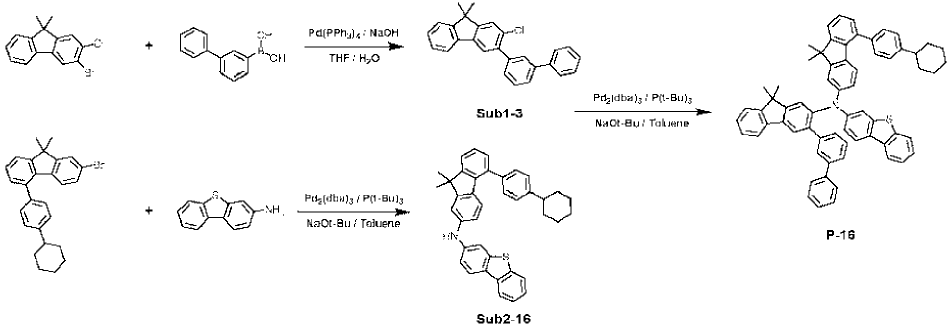

### 1) Synthesis of Sub1-3

3-bromo-2-chloro-9,9-dimethyl-9H-fluorene (50.0 g, 162.5 mmol) was dissolved in THF (542 mL), and then [1,1'-biphenyl]-3-ylboronic acid (35.4 g, 178.8 mmol), NaOH (19.5 g, 487.6 mmol), Pd(PPh₃)₄ (9.39 g, 8.13 mmol), and water (181 ml) were added thereto. The reaction was carried out in the same manner as in the preparation of Sub1-1 to obtain 46.4 g of the product (yield: 75%).

### 2) Synthesis of Sub2-16

2-bromo-5-(4-cyclohexylphenyl)-9,9-dimethyl-9H-fluorene (50.0 g, 115.9 mmol) was dissolved in toluene (386 mL), and then dibenzo[b,d]thiophen-3-amine (27.7 g, 139.1 mmol), Pd₂(dba)₃ (3.48 g, 3.48 mmol), P(t-Bu)₃ (1.41 g, 6.95 mmol), and NaOt-Bu (22.3 g, 231.8 mmol) were added thereto. The reaction was carried out in the same manner as in the preparation of Sub2-1 to obtain 45.2 g of the product (yield: 71%).

### 3) Synthesis of P-16

Sub1-3 (20.0 g, 65.0 mmol) was dissolved in toluene (325 mL), and then Sub2-16 (35.7 g, 65.0 mmol), Pd₂(dba)₃ (1.79 g, 1.95 mmol), P(t-Bu)₃ (0.79 g, 3.90 mmol), and NaOt-Bu (18.7 g, 195.1 mmol) were added thereto. The reaction was carried out in the same manner as in the preparation of P-1 to obtain 42.4 g of the product (yield: 73%).

### 4. Synthesis Example of P-22

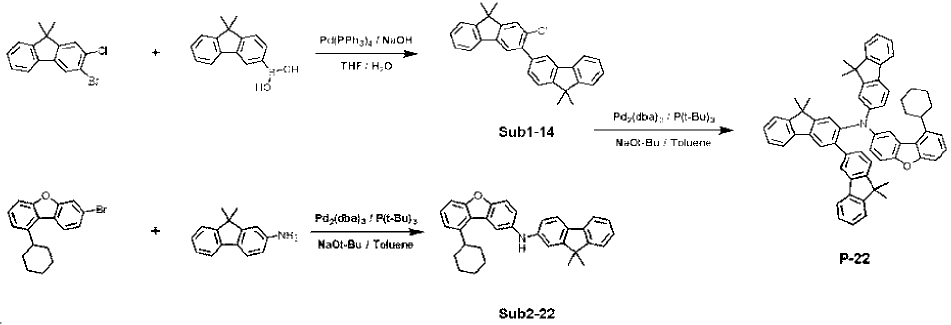

### 1) Synthesis of Sub1-14

3-bromo-2-chloro-9,9-dimethyl-9H-fluorene (50.0 g, 162.5 mmol) was dissolved in THF (542 mL), and then (9,9-dimethyl-9H-fluoren-3-yl)boronic acid (42.6 g, 178.8 mmol), NaOH (19.5 g, 487.6 mmol), Pd(PPh₃)₄ (9.39 g, 8.13 mmol), and water (181 ml) were added thereto. The reaction was carried out in the same manner as in the preparation of Sub1-1 to obtain 56.1 g of the product (yield: 82%).

### 2) Synthesis of Sub2-22

7-bromo-1-cyclohexyldibenzo[b,d]furan (50.0 g, 151.9 mmol) was dissolved in toluene (506 mL), and then 9,9-dimethyl-9H-fluoren-2-amine (38.1 g, 182.2 mmol), Pd₂(dba)₃ (4.17 g, 4.56 mmol), P(t-Bu)₃ (1.84 g, 9.11 mmol), and NaOt-Bu (29.2 g, 303.7 mmol) were added thereto. The reaction was carried out in the same manner as in the preparation of Sub2-1 to obtain 52.8 g of the product (yield: 76%).

### 3) Synthesis of P-22

Sub1-14 (30.0 g, 71.3 mmol) was dissolved in toluene (380 mL), and then Sub2-22 (32.6 g, 71.3 mmol), Pd₂(dba)₃ (1.96 g, 2.14 mmol), P(t-Bu)₃ (0.87 g, 2.14 mmol), and NaOt-Bu (20.5 g, 213.8 mmol) were added thereto. The reaction was carried out in the same manner as in the preparation of P-1 to obtain 47.4 g of the product (yield: 79%).

### 5. Synthesis Example of P-30

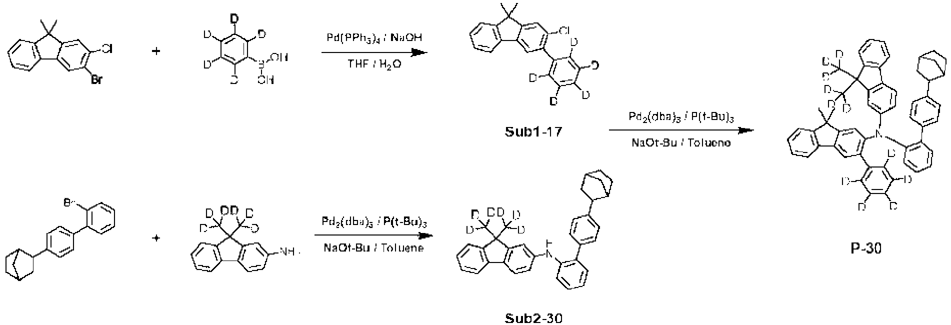

### 1) Synthesis of Sub1-17

3-bromo-2-chloro-9,9-dimethyl-9H-fluorene (50.0 g, 162.5 mmol) was dissolved in THF (542 mL), and then (phenyl-d5)boronic acid (22.7 g, 178.8 mmol), NaOH (19.5 g, 487.6 mmol), Pd(PPh₃)₄ (9.39 g, 8.13 mmol), and water (181 ml) were added thereto. The reaction was carried out in the same manner as in the preparation of Sub1-1 to obtain 41.8 g of the product (yield: 83%).

### 2) Synthesis of Sub2-30

2-(2'-bromo-[1,1'-biphenyl]-4-yl)bicyclo[2.2.1]heptane (50.0 g, 152.8 mmol) was dissolved in toluene (509 mL), and then 9,9-bis(methyl-d3)-9H-fluoren-2-amine (39.5 g, 183.3 mmol), Pd₂(dba)₃ (4.20 g, 4.58 mmol), P(t-Bu)₃ (1.85 g, 9.17 mmol), and NaOt-Bu (29.4 g, 305.6 mmol) were added thereto. The reaction was carried out in the same manner as in the preparation of Sub2-1 to obtain 55.0 g of the product (yield: 78%).

### 3) Synthesis of P-30

Sub1-17 (30.0 g, 96.8 mmol) was dissolved in toluene (484 mL), and then Sub2-30 (44.7 g, 96.8 mmol), Pd₂(dba)₃ (2.66 g, 2.90 mmol), P(t-Bu)₃ (1.18 g, 5.81 mmol), and NaOt-Bu (27.9 g, 290.5 mmol) were added thereto. The reaction was carried out in the same manner as in the preparation of P-1 to obtain 56.9 g of the product (yield: 80%).

### 6. Synthesis Example of P-44

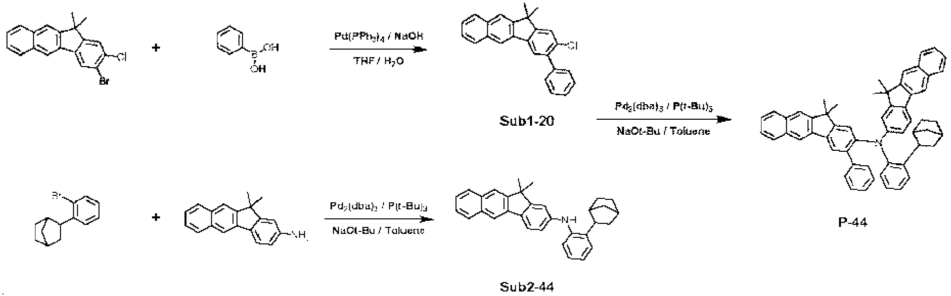

### 1) Synthesis of Sub1-20

3-bromo-2-chloro-11,11-dimethyl-11H-benzo[b]fluorene (50.0 g, 139.8 mmol) was dissolved in THF (466 mL), and then phenylboronic acid (18.7 g, 153.8 mmol), NaOH (16.8 g, 419.4 mmol), Pd(PPh₃)₄ (8.08 g, 6.99 mmol), and water (155 ml) were added thereto. The reaction was carried out in the same manner as in the preparation of Sub1-1 to obtain 42.2 g of the product (yield: 85%).

### 2) Synthesis of Sub2-44

2-(2-bromophenyl)bicyclo[2.2.1]heptane (50.0 g, 199.1 mmol) was dissolved in toluene (664 mL), and then 11,11-dimethyl-11H-benzo[b]fluoren-2-amine (62.0 g, 238.9 mmol), Pd₂(dba)₃ (5.47 g, 5.97 mmol), P(t-Bu)₃ (2.42 g, 11.94 mmol), and NaOt-Bu (38.3 g, 398.1 mmol) were added thereto. The reaction was carried out in the same manner as in the preparation of Sub2-1 to obtain 65.0 g of the product (yield: 76%).

### 3) Synthesis of P-44

Sub1-20 (30.0 g, 84.5 mmol) was dissolved in toluene (423 mL), and then Sub2-44 (36.3 g, 84.5 mmol), Pd₂(dba)₃ (2.32 g, 2.54 mmol), P(t-Bu)₃ (1.03 g, 5.07 mmol), and NaOt-Bu (24.4 g, 253.6 mmol) were added thereto. The reaction was carried out in the same manner as in the preparation of P-1 to obtain 51.2 g of the product (yield: 81%).

### 7. Synthesis Example of P-49

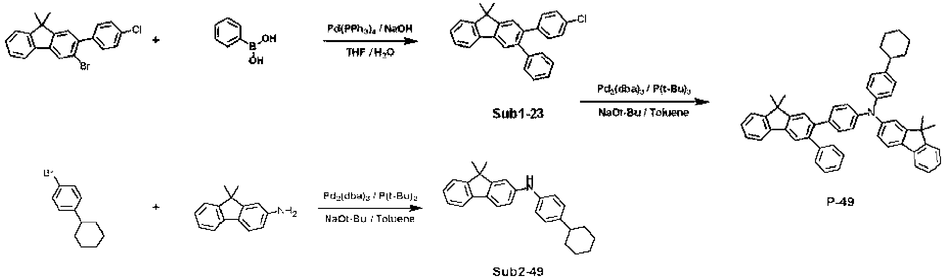

### 1) Synthesis of Sub1-23

3-bromo-2-(4-chlorophenyl)-9,9-dimethyl-9H-fluorene (50.0 g, 130.3 mmol) was dissolved in THF (434 mL), and then phenylboronic acid (17.5 g, 143.3 mmol), NaOH (15.6 g, 390.9 mmol), Pd(PPh₃)₄ (7.53 g, 6.52 mmol), and water (145 ml) were added thereto. The reaction was carried out in the same manner as in the preparation of Sub1-1 to obtain 41.2 g of the product (yield: 83%).

### 2) Synthesis of Sub2-49

bromo-4-cyclohexylbenzene (50.0 g, 209.1 mmol) was dissolved in toluene (697 mL), and then 9,9-dimethyl-9H-fluoren-2-amine (52.5 g, 250.9 mmol), Pd₂(dba)₃ (5.74 g, 6.27 mmol), P(t-Bu)₃ (2.54 g, 12.54 mmol), and NaOt-Bu (40.2 g, 418.1 mmol) were added thereto. The reaction was carried out in the same manner as in the preparation of Sub2-1 to obtain 62.2 g of the product (yield: 81%).

### 3) Synthesis of P-49

Sub1-23 (30.0 g, 78.8 mmol) was dissolved in toluene (394 mL), and then Sub2-49 (28.9 g, 78.8 mmol), Pd₂(dba)₃ (2.16 g, 2.36 mmol), P(t-Bu)₃ (0.96 g, 4.73 mmol), and NaOt-Bu (22.7 g, 236.3 mmol) were added thereto. The reaction was carried out in the same manner as in the preparation of P-1 to obtain 46.0 g of the product (yield: 82%).

### 8. Synthesis Example of P-74

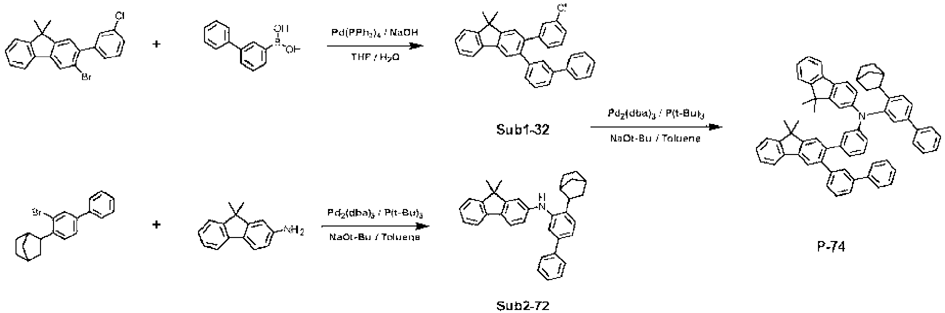

### 1) Synthesis of Sub1-32

3-bromo-2-(3-chlorophenyl)-9,9-dimethyl-9H-fluorene (50.0 g, 130.3 mmol) was dissolved in THF (434 mL), and then [1,1'-biphenyl]-3-ylboronic acid (28.4 g, 198.03 mmol), NaOH (15.6 g, 390.9 mmol), Pd(PPh₃)₄ (7.53 g, 6.52 mmol), and water (145 ml) were added thereto. The reaction was carried out in the same manner as in the preparation of Sub1-1 to obtain 44.7 g of the product (yield: 75%).

### 2) Synthesis of Sub2-72

2-(3-bromo-[1,1'-biphenyl]-4-yl)bicyclo[2.2.1]heptane (50.0 g, 152.8 mmol) was dissolved in toluene (509 mL), and then 9,9-dimethyl-9H-fluoren-2-amine (38.4 g, 183.3 mmol), Pd₂(dba)₃ (4.20 g, 4.58 mmol), P(t-Bu)₃ (1.85 g, 9.17 mmol), and NaOt-Bu (29.4 g, 305.6 mmol) were added thereto. The reaction was carried out in the same manner as in the preparation of Sub2-1 to obtain 57.8 g of the product (yield: 83%).

### 3) Synthesis of P-74

Sub1-32 (30.0 g, 78.2 mmol) was dissolved in toluene (391 mL), and then Sub2-72 (35.6 g, 78.2 mmol), Pd₂(dba)₃ (2.15 g, 2.35 mmol), P(t-Bu)₃ (0.95 g, 4.69 mmol), and NaOt-Bu (22.5 g, 234.6 mmol) were added thereto. The reaction was carried out in the same manner as in the preparation of P-1 to obtain 48.6 g of the product (yield: 71%).

### 9. Synthesis Example of P-86

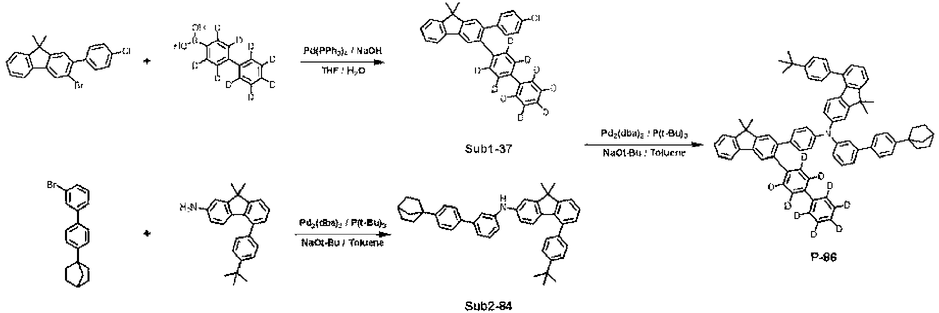

### 1) Synthesis of Sub1-37

3-bromo-2-(4-chlorophenyl)-9,9-dimethyl-9H-fluorene (50.0 g, 130.3 mmol) was dissolved in THF (434 mL), and then ([1,1'-biphenyl]-4-yl-d9)boronic acid (29.7 g, 143.3 mmol), NaOH (15.6 g, 390.9 mmol), Pd(PPh₃)₄ (7.53 g, 6.52 mmol), and water (145 ml) were added thereto. The reaction was carried out in the same manner as in the preparation of Sub1-1 to obtain 46.8 g of the product (yield: 77%).

### 2) Synthesis of Sub2-84

1-(3'-bromo-[1,1'-biphenyl]-4-yl)bicyclo[2.2.1]heptane (50.0 g, 152.8 mmol) was dissolved in toluene (509 mL), and then 5-(4-(tert-butyl)phenyl)-9,9-dimethyl-9H-fluoren-2-amine (62.6 g, 183.3 mmol), Pd₂(dba)₃ (4.20 g, 4.58 mmol), P(t-Bu)₃ (1.85 g, 9.17 mmol), and NaOt-Bu (29.4 g, 305.6 mmol) were added thereto. The reaction was carried out in the same manner as in the preparation of Sub2-1 to obtain 67.4 g of the product (yield: 75%).

### 3) Synthesis of P-86

Sub1-37(30.0 g, 64.4 mmol) was dissolved in toluene (322 mL), and then Sub2-84 (37.8 g, 64.4 mmol), Pd₂(dba)₃ (1.77 g, 1.93 mmol), P(t-Bu)₃ (0.78 g, 3.86 mmol), and NaOt-Bu (18.6 g, 193.1 mmol) were added thereto. The reaction was carried out in the same manner as in the preparation of P-1 to obtain 50.4 g of the product (yield: 77%).

The FD-MS values of the compounds P-1 to P-100 of the present invention, prepared according to the above Synthesis Examples, are shown in Table 3 below.

**[Table 3]**

| Compound | FD-MS | Compound | FD-MS |
|---|---|---|---|
| P-1 | m/z=711.39(C₅₄H₄₉N=711.99) | P-2 | m/z=799.42(C₆₁H₅₃N=800.1) |
| P-3 | m/z=799.42(C₆₁H₅₃N=800.1) | P-4 | m/z=787.42(C₆₀H₅₃N=788.09) |
| P-5 | m/z=773.4(C₅₉H₅₁N=774.06) | P-6 | m/z=773.4(C₅₉H₅₁N=774.06) |
| P-7 | m/z=811.42(C₆₂H₅₃N=812.11) | P-8 | m/z=811.42(C₆₂H₅₃N=812.11) |
| P-9 | m/z=951.48(C₇₃H₆₁N=952.3) | P-10 | m/z=913.43(C₆₉H₅₅NO=914.2) |
| P-11 | m/z=925.43(C₇₀H₅₅NO=926.22) | P-12 | m/z=925.43(C₇₀H₅₅NO=926.22) |
| P-13 | m/z=837.4(C₆₃H₅₁NO=838.11) | P-14 | m/z=865.37(C₆₄H₅₁NS=866.18) |
| P-15 | m/z=867.39(C₆₄H₅₃NS=868.2) | P-16 | m/z=893.41(C₆₆H₅₅NS=894.23) |
| P-17 | m/z=3861.73(C₂₈₈H₂₂₆N₇₀S₂₃=3865.14) | P-18 | m/z=954.4(C₇₀H₅₄N₂S=955.28) |
| P-19 | m/z=1077.5(C₈₁H₆₃N₃=1078.42) | P-20 | m/z=963.48(C₇₄H₆₁N=964.31) |
| P-21 | m/z=1217.55(C₉₃H₇₁ NO=1218.6) | P-22 | m/z=841.43(C₆₃H₅₅NO=842.14) |
| P-23 | m/z=919.38(C₆₇H₅₃NOS=920.23) | P-24 | m/z=1111.48(C₈₂H₆₅NOS=1112.49) |
| P-25 | m/z=1210.53(C₉₀H₇₀N₂S=1211.62) | P-26 | m/z=1061.55(C₈₀H₇₁NO=1062.45) |
| P-27 | m/z=931.51(C₇₁ H₆₅N=932.31) | P-28 | m/z=959.54(C₇₃H₆₉N=960.36) |
| P-29 | m/z=756.45(C₅₇H₄₈D₅N=757.09) | P-30 | m/z=734.46(C₅₅H₃₈D₁₁N=735.07) |
| P-31 | m/z=810.49(C₆₁ H₄₂D₁₁N=811.17) | P-32 | m/z=777.34(C₅₇H₄₇NS=778.07) |
| P-33 | m/z=782.46(C₅₉H₄₂D₉N=783.12) | P-34 | m/z=697.37(C₅₃H₄₇N=697.97) |
| P-35 | m/z=685.37(C₅₂H₄₇N=685.96) | P-36 | m/z=779.44(C₅₉H₄₅D₆N=780.1) |
| P-37 | m/z=799.42(C₆₁H₅₃N=800.1) | P-38 | m/z=884.51(C₆₇H₄₈D₉N=885.25) |
| P-39 | m/z=794.46(C₆₀H₄₆D₇N=795.13) | P-40 | m/z=805.46(C₆₁H₄₇D₆N=806.14) |
| P-41 | m/z=837.36(C₆₂H₄₇NO₂=838.06) | P-42 | m/z=909.31(C₆₄H₄₇NOS₂=910.21) |
| P-43 | m/z=923.53(C₇₀H₅₇D₆N=924.32) | P-44 | m/z=747.39(C₅₇H₄₉N=748.03) |
| P-45 | m/z=832.44(C₆₂H₄₀D₉NO=833.13) | P-46 | m/z=647.43(C₄₈H₃₃D₁₂N=647.97) |
| P-47 | m/z=666.47(C₄₉H₂₆D₁₉N=667.02) | P-48 | m/z=742.51(C₅₅H₃₀D₁₉N=743.12) |
| P-49 | m/z=711.39(C₅₄H₄₉N=711.99) | P-50 | m/z=799.42(C₆₁H₅₃N=800.1) |
| P-51 | m/z=723.39(C₅₅H₄₉N=724) | P-52 | m/z=863.45(C₆₆H₅₇N=864.19) |
| P-53 | m/z=799.42(C₆₁H₅₃N=800.1) | P-54 | m/z=951.48(C₇₃H_{6\1} N=952.3) |
| P-55 | m/z=837.43(C₆₄H₅₅N=838.15) | P-56 | m/z=875.45(C₆₇H₅₇N=876.2) |
| P-57 | m/z=875.45(C₆₇H₅₇N=876.2) | P-58 | m/z=837.4(C₆₃H₅₁ NO=838. 11) |
| P-59 | m/z=829.43(C₆₂H₅₅NO=830.13) | P-60 | m/z=773.37(C₅₈H₄₇NO=774.02) |
| P-61 | m/z=837.4(C₆₃H₅₁NO=838.11) | P-62 | m/z=865.37(C₆₄H₅₁NS=866.18) |
| P-63 | m/z=865.37(C₆₄H₅₁NS=866.18) | P-64 | m/z=853.37(C₆₃H₅₁NS=854.17) |
| P-65 | m/z=924.44(C₇₀H₅₆N₂=925.23) | P-66 | m/z=865.37(C₆₄H₅₁NS=866.18) |
| P-67 | m/z=988.48(C₇₅H₆₀N₂=989.32) | P-68 | m/z=999.48(C₇₇H₆₁N=1000.34) |
| P-69 | m/z=1089.49(C₈₃H₆₃NO=1090.42) | P-70 | m/z=877.43(C₆₆H₅₅NO=878.17) |
| P-71 | m/z=879.35(C₆₄H₄₉NOS=880.16) | P-72 | m/z=117.53(C₈₈H₆₉NO₂=1172.52) |
| P-73 | m/z=999.39(C₇₂H₅₇NS₂=1 000.37) | P-74 | m/z=875.45(C₆₇H₅₇N=876.2) |
| P-75 | m/z=987.57(C₇₅H₇₃N=988.42) | P-76 | m/z=919.51(C₇₀H₆₅N=920.3) |
| P-77 | m/z=846.5(C₆₄H₅₄D₅N=847.21) | P-78 | m/z=966.57(C₇₃H₄₆D₁₅N=967.39) |
| P-79 | m/z=886.52(C₆₇H₄₆D₁₁N=887.27) | P-80 | m/z=898.44(C₆₆H₅₀D₅NS=899.26) |
| P-81 | m/z=823.42(C₆₃H₅₃N=824.12) | P-82 | m/z=823.42(C₆₃H₅₃N=824.12) |
| P-83 | m/z=884.51(C₆₇H₄₈D₉N=885.25) | P-84 | m/z=855.47(C₆₅H₄₉D₆N=856.2) |
| P-85 | m/z=799.42(C₆₁H₅₃N=800.1) | P-86 | m/z=1016.6(C₇₇H₆₀D₉N=1017.46) |
| P-87 | m/z=794.46(C₆₀H₄₆D₇N=795.13) | P-88 | m/z=937.55(C₇₁H₅₉D₆N=938.34) |
| P-89 | m/z=939.41(C₇₀H₅₃NO₂=940.2) | P-90 | m/z=985.34(C₇₀H₅₁NOS₂=986.3) |
| P-91 | m/z=891.48(C₆₈H₆₁N=892.24) | P-92 | m/z=899.45(C₆₉H₅₇N=900.22) |
| P-93 | m/z=879.44(C₆₆H₅₇NO=880.19) | P-94 | m/z=723.46(C₅₄H₃₇D₁₂N=724.07) |
| P-95 | m/z=818.54(C₆₁H₃₄D₁₉N=819.22) | P-96 | m/z=818.54(C₆₁H₃₄D₁₉N=819.22) |
| P-97 | m/z=659.32(C₄₉H₄₁NO=659.87) | P-98 | m/z=793.46(C₆₀H₅₉N=794.14) |
| P-99 | m/z=635.36(C₄₈H₄₅N=635.90) | P-100 | m/z=889.61(C₆₇H₄₅D₁₈N=900.36) |

Although synthesis examples of compounds represented by Formula 1 have been described above, these reactions are all based on Buchwald-Hartwig cross-coupling, Miyaura borylation, Suzuki cross-coupling, Intramolecular acid-induced cyclization (J. Mater. Chem. 1999, 9, 2095), Pd(II)-catalyzed oxidative cyclization (Org. Lett. 2011, 13, 5504), and PPh₃-mediated reductive cyclization (J. Org. Chem. 2005, 70, 5014), etc. It will be readily understood by those skilled in the art that these reactions can proceed even when substituents other than those specifically described in the synthesis examples, as defined in Formula 1, are introduced.

### Manufacturing and Evaluation of organic electronic device

### [Test Example 1] Red organic electroluminescent device (emission auxiliary layer)

A hole injection layer having a thickness of 10 nm was formed on an ITO layer (anode) formed on a glass substrate by vacuum-depositing N-([1,1'-biphenyl]-4-yl)-9,9-dimethyl-N-(4-(9-phenyl-9H-carbazol-3-yl)phenyl)-9H-fluoren-2-amine (hereinafter referred to as Compound A) and 4,4',4"-((1E,1'E,1"E)-cyclopropane-1,2,3-triylidenetris(cyanomethaneylylidene))tris(2,3,5,6-tetrafluorobenzonitrile) (hereinafter referred to as Compound B). At this time, Compound B was doped such that the weight ratio of Compound A to Compound B was 98:2.

Thereafter, a hole transport layer having a thickness of 110 nm was formed on the hole injection layer by vacuum deposition of Compound A.

Thereafter, an emission auxiliary layer having a thickness of 10 nm was formed on the hole transport layer by vacuum-depositing the compound P-1 of the present invention.

Thereafter, an emission layer having a thickness of 30 nm was formed on the emission auxiliary layer by vacuum-depositing 14-(4-phenylquinazolin-2-yl)-14H-benzo[c]benzo[4,5]thieno[2,3-a]carbazole as the host, and bis(1-phenylisoquinolyl)iridium(III) acetylacetonate (hereinafter abbreviated as "(piq)₂Ir(acac)") as the dopant. At this time, the dopant was doped such that the weight ratio of the host to the dopant was 95:5.

Thereafter, a hole blocking layer having a thickness of 10 nm was formed on the emission layer by vacuum-depositing 2-(4'-(9,9-dimethyl-9H-fluoren-2-yl)-[1,1'-biphenyl]-3-yl)-4,6-diphenyl-1,3,5-triazine.

Thereafter, an electron transport layer having a thickness of 30 nm was formed on the hole blocking layer by vacuum-depositing a mixture of 2,7-bis(4-(4,6-diphenyl-1,3,5-triazin-2-yl)phenyl)naphthalene and (8-quinolinolato)lithium in a weight ratio of 5:5.

Thereafter, an electron injection layer having a thickness of 0.2 nm was formed on the electron transport layer by vacuum-depositing (8-quinolinolato)lithium, and subsequently, Al was deposited to form a cathode having a thickness of 150 nm.

### [Test Example 2] to [Test Example 20]

An organic electroluminescent device was fabricated in the same manner as in the Test Example 1, except that Compound P-1 of the present invention was replaced with the compound of the present invention shown in Table 4 below as the emission auxiliary layer material.

### [Comparative Example 1] to [Comparative Example 3]

An organic electroluminescent device was fabricated in the same manner as in the Test Example 1, except that Compound P-1 of the present invention was replaced with one selected from Comparative Compounds A to C as the emission auxiliary layer material.

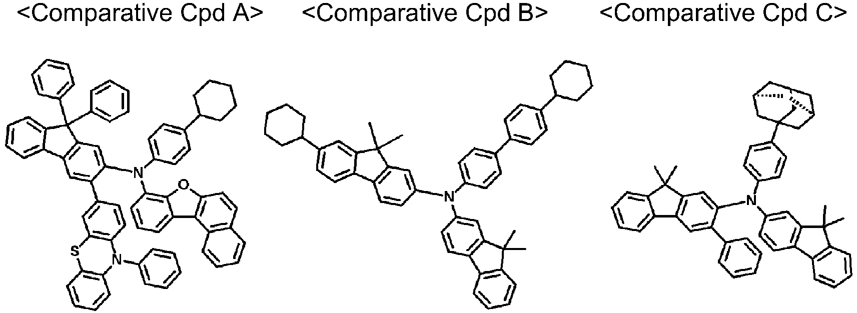

A forward bias direct current voltage was applied to the organic electroluminescent devices prepared according to Examples 1 to 20 and Comparative Examples 1 to 3 of the present invention, and electroluminescent (EL) characteristics were measured using a PR-650 manufactured by Photo Research. In addition, T95 was measured at a luminance of 2,500 cd/m² using a lifetime measurement apparatus manufactured by Mc Science. The measurement results are shown in Table 4 below.

Such measurement apparatus is not affected by day-to-day variations in deposition rate, vacuum quality, or other parameters, and thus allows evaluation of new performance in comparison with comparative compounds under identical conditions. During evaluation, each batch included four identically prepared OLEDs including those containing comparative compounds, and since a total of 12 OLEDs from three batches were evaluated, the obtained experimental results have statistical significance.

**[Table 4]**

| | Compound | Driving Voltage (V) | Current Density (mA/cm²) | Brightness (cd/m²) | Efficiency (cd/A) | T(95) |
|---|---|---|---|---|---|---|
| comp.Ex1 | Comparative compound A | 5.5 | 16.9 | 2500.0 | 14.8 | 81.1 |
| comp.Ex2 | Comparative compound B | 5.2 | 13.2 | 2500.0 | 18.9 | 91.6 |
| comp.Ex3 | Comparative compound C | 5.1 | 14.3 | 2500.0 | 17.5 | 89.4 |
| Test Ex.1 | P-1 | 4.2 | 6.5 | 2500.0 | 38.4 | 123.7 |
| Test Ex.2 | P-6 | 4.1 | 6.8 | 2500.0 | 36.6 | 124.8 |
| Test Ex.3 | P-11 | 4.5 | 7.4 | 2500.0 | 34.0 | 117.0 |
| Test Ex.4 | P-23 | 4.5 | 7.4 | 2500.0 | 33.8 | 117.8 |
| Test Ex.5 | P-27 | 4.2 | 6.7 | 2500.0 | 37.1 | 127.6 |
| Test Ex.6 | P-36 | 4.2 | 6.7 | 2500.0 | 37.5 | 121.1 |
| Test Ex.7 | P-41 | 4.4 | 7.9 | 2500.0 | 31.8 | 112.9 |
| Test Ex.8 | P-44 | 4.3 | 6.4 | 2500.0 | 39.3 | 129.1 |
| Test Ex.9 | P-49 | 4.1 | 7.0 | 2500.0 | 35.9 | 122.5 |
| Test Ex.10 | P-52 | 4.3 | 6.7 | 2500.0 | 37.1 | 121.5 |
| Test Ex.11 | P-58 | 4.5 | 7.8 | 2500.0 | 32.0 | 114.4 |
| Test Ex.12 | P-63 | 4.4 | 7.4 | 2500.0 | 33.8 | 120.8 |
| Test Ex.13 | P-80 | 4.5 | 8.1 | 2500.0 | 30.9 | 110.5 |
| Test Ex.14 | P-81 | 4.2 | 7.1 | 2500.0 | 35.4 | 123.0 |
| Test Ex.15 | P-90 | 4.4 | 7.8 | 2500.0 | 32.2 | 111.7 |
| Test Ex.16 | P-94 | 4.2 | 6.6 | 2500.0 | 37.9 | 129.3 |
| Test Ex.17 | P-97 | 4.4 | 8.2 | 2500.0 | 30.6 | 117.5 |
| Test Ex.18 | P-98 | 4.2 | 6.6 | 2500.0 | 37.6 | 128.9 |
| Test Ex.19 | P-99 | 4.1 | 6.3 | 2500.0 | 39.6 | 126.8 |
| Test Ex.20 | P-100 | 4.1 | 6.7 | 2500.0 | 37.5 | 123.4 |

As can be seen from Table 4, when the compound of the present invention is used as an emission auxiliary layer material, the organic electroluminescent device exhibits a remarkably lower driving voltage and significantly improved efficiency and lifetime, compared with the cases where any one of Comparative Compounds A to C is used (Comparative Examples 1 to 3). First, the structural differences between the compound of the present invention and Comparative Compound A, and the effect of the resulting difference in reorganization energy on the device characteristics, will be described.

Comparative Compound A is similar to the compound of the present invention in that an amino group is bonded to the 2-position of the fluorene moiety. However, it differs in that the substituents at the 9- and 9'-positions of the fluorene moiety are phenyl groups rather than alkyl groups, and that a heterocyclic group, rather than an aryl group, is substituted at the 3-position of the fluorene moiety.

In order to examine the effect of these structural differences on the reorganization energy of the compounds, the reorganization energies of Comparative Compound A and Compound P-97 of the present invention, which has a structure similar thereto, were calculated using the DFT method (B3LYP/6-31G(d)) of the Gaussian program. The results are shown in Table 5 below. As used herein, the RE value refers to the REₕₒₗₑ value.

**[Table 5]**

| Compound | HOMO (eV) | Reorganization Energy (RE) |
|---|---|---|
| Comparative compound A | -4.760 | 0.226 |
| P-97 | -4.868 | 0.193 |

As can be seen from Table 5, Compound P-97 of the present invention, which has a structure similar to that of Comparative Compound A, has a lower RE value than Comparative Compound A. Accordingly, when the compound of the present invention having the lower RE value is used as an emission auxiliary layer material, it exhibits higher hole mobility than Comparative Compound A, as demonstrated by its superior hole-only device (HOD) characteristics, resulting in significantly improved hole transport and hole injection. Consequently, the driving voltage is reduced and the charge balance in the emission layer is maximized, whereby the driving voltage and efficiency of the device are considered to be remarkably improved. Next, the structural differences between the compound of the present invention and Comparative Compound B, and the effect of the resulting difference in the T1 energy level on the device characteristics, will be described.

Comparative Compound B is similar to the compound of the present invention in that one of the substituents on the amino group is an alicyclic group. However, it differs in that no substituent corresponding to Ar^{a} of the present invention is present at the 3-position of the fluorene moiety.

In order to examine the effect of these structural differences on the T1 energy level of the compounds, the T1 energy levels of Comparative Compound B and Compound P-98 of the present invention, which has a structure similar thereto, were calculated using the DFT method (B3LYP/6-31G(d)) of the Gaussian program. The results are shown in Table 6 below.

**[Table 6]**

| Compound | T1 (eV) |
|---|---|
| Comparative compound B | 2.597 |
| P-98 | 2.645 |

As can be seen from Table 6, the compound of the present invention has a significantly higher T1 energy level than Comparative Compound B. Accordingly, when the compound of the present invention having the higher T1 energy level is used as an emission auxiliary layer material, it can effectively block triplet excitons transferred from the emission layer, as compared with Comparative Compound B. As a result, the efficiency and lifetime of the device are considered to be remarkably improved. Finally, the structural differences between the compound of the present invention and Comparative Compound C, and the effect of the resulting difference in hole neutral stabilization energy on the device characteristics, will be described.

Comparative Compound C is similar to the compound of the present invention in that one of the substituents on the amino group is an alicyclic group and an aryl group is substituted at the 3-position of the fluorene moiety. However, it differs in the type of the alicyclic group from the compound of the present invention.

In order to examine the effect of these structural differences on the hole neutral stabilization energy (unit: eV; hereinafter abbreviated as "HNSE") of the compounds, the HNSE values of Comparative Compound C and Compound P-99 of the present invention, which has a structure similar thereto, were calculated by molecular simulation using the Schrödinger Materials Science Suite 4.9.128. The results are shown in Table 7 below.

**[Table 7]**

| Compound | HNSE(eV) |
|---|---|
| Comparative compound C | 0.070 |
| P-99 | 0.060 |

As can be seen from Table 7, the compound P-99 of the present invention has a lower HNSE value than Comparative Compound C. This indicates that, when the compound of the present invention is used as an emission auxiliary layer material, it can reach a stable state more rapidly with less energy than Comparative Compound C after receiving holes from the hole transport layer or after transferring holes to the emission layer within the device. Accordingly, when the compound of the present invention is used as an emission auxiliary layer material, the lifetime of the device is considered to be remarkably improved.
As can be seen from Tables 5 to 7, even among compounds having similar structures, the compounds of the present invention, which satisfy all of the combined factors, such as the types of substituents and the substitution positions of the substituents, exhibit remarkably superior performance in an organic electronic device compared with the comparative compounds. Accordingly, it can be understood that the compounds of the present invention also exhibit remarkably superior performance in an organic electronic device compared with other compounds having similar structures that are not specifically described herein.

Based on these results, it can be seen that, even when compounds having similar structures are used as the emission auxiliary layer material, the properties of the compounds, such as hole transport characteristics, photoluminescence efficiency, energy levels, hole injection and mobility characteristics, charge balance between holes and electrons, packing density, and intermolecular distance, may vary depending on a combination of factors including the type of substituents and the substitution positions thereof. Accordingly, it can be seen that such differences may result in significant differences in device performance.

In the case of an emission auxiliary layer, the interaction with a hole transport layer and an emission layer (host) should be taken into consideration. Accordingly, even if compounds having a similar core structure are employed, it would be extremely difficult for a person skilled in the art to predict the characteristics exhibited by an emission auxiliary layer comprising the compounds of the present invention.

In the foregoing evaluation of device fabrication, the device characteristics were described with respect to devices in which the compounds of the present invention were applied only to an emission auxiliary layer. However, the compounds of the present invention may also be applied to a hole transport layer, or to both a hole transport layer and an emission auxiliary layer.

The foregoing description is merely illustrative of the present invention and various modifications may be made by those of ordinary skill in the art without departing from the essential characteristics of the invention. The scope of protection of the present invention should be interpreted based on the following claims, and all technical equivalents thereof should be construed as falling within the scope of the invention.

## Claims

1. A compound of Formula 1: 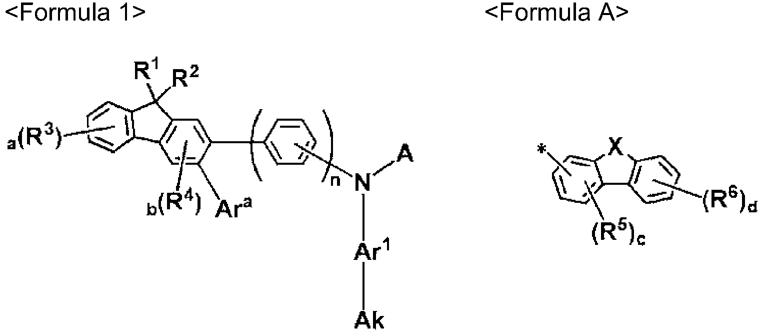 in the above Formula,
A is Formula A,
X is C(R^{a})(R^{b}), O, S or N(R^{c}),
Ak is selected from the group consisting of Formula Ak-1 to Formula Ak-3, 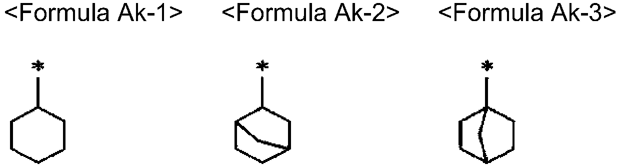
in Formula Ak-1 to Ak-3, hydrogen may be replaced with deuterium,
Ar¹ is selected from the group consisting of a C₆-C₆₀ arylene group; a fluorenylene group; a C₂-C₆₀ heterocyclic group containing at least one heteroatom of O, N, S, Si and P; a fused ring of a C₆-C₆₀ aromatic ring and a C₃-C₆₀ alicyclic group; and a combination thereof,
Ar^{a} is a C₆-C₆₀ aryl group or a fluorenyl group,
R¹ and R² is a C₁-C₂₀ alkyl group, wherein the hydrogen of the alkyl group may be replaced with deuterium,
R³ to R⁶ are each independently selected from the group consisting of hydrogen; deuterium; halogen; a cyano group; a nitro group; a silane group; a C₆-C₆₀ aryl group; a fluorenyl group; a C₂-C₆₀ heterocyclic group containing at least one heteroatom selected from O, N, S, Si, and P; a C₃-C₆₀ alicyclic group; a fused ring group of a C₆-C₆₀ aromatic ring and a C₃-C₆₀ alicyclic ring; a C₁-C₂₀ alkyl group; a C₂-C₂₀ alkenyl group; a C₂-C₂₀ alkynyl group; a C₁-C₂₀ alkoxy group; and a C₆-C₆₀ aryloxy group, and adjacent groups may be bonded to each other to form a ring,
a and d are each an integer of 0 to 4, b is an integer of 0 to 2, c is an integer of 0 to 3, n is an integer of 0 or 1,
R^{a} and R^{b} are each independently selected from the group consisting of hydrogen; deuterium; halogen; a cyano group; a nitro group; a C₆-C₆₀ aryl group; a fluorenyl group; a C₂-C₆₀ heterocyclic group containing at least one heteroatom selected from O, N, S, Si, and P; a C₃-C₆₀ alicyclic group; a fused ring group of a C₆-C₆₀ aromatic ring and a C₃-C₆₀ alicyclic ring; a C₁-C₂₀ alkyl group; a C₂-C₂₀ alkenyl group; a C₂-C₂₀ alkynyl group; a C₁-C₂₀ alkoxy group; and a C₆-C₆₀ aryloxy group,
R^{c} is selected from the group consisting of a C₆-C₆₀ aryl group; a fluorenyl group; a C₂-C₆₀ heterocyclic group containing at least one heteroatom of O, N, S, Si and P; and a fused ring of a C₆-C₆₀ aromatic ring and a C₃-C₆₀ alicyclic group, and
Ar¹, Ar^{a}, R³ to R⁶, R^{a} to R^{c} may be each substituted with one or more substituents selected from the group consisting of deuterium; halogen; a silane group unsubstituted or substituted with a C₁-C₂₀ alkyl group or a C₆-C₂₀ aryl group; a phosphine oxide substituted or unsubstituted with a C₁-C₂₀ alkyl group or a C₆-C₂₀ aryl group; a cyano group; a nitro group; a C₁-C₂₀ alkylthio group; a C₁-C₂₀ alkoxy group; a C₆-C₃₀ aryloxy group; a C₆-C₃₀ arylthio group; a C₁-C₂₀ alkyl group; a C₂-C₂₀ alkenyl group; a C₂-C₂₀ alkynyl group; a C₆-C₃₀ aryl group; a deuterium-substituted C₆-C₃₀ aryl group; a C₃-C₃₀ alicyclic group; a fused ring of a C₆-C₃₀ aromatic ring and a C₃-C₃₀ alicyclic group; and a C₂-C₃₀ heterocyclic group comprising at least one heteroatom selected from the group consisting of O, N, S, Si and P, and the adjacent substituents may be bonded to each other to form a ring (provided that the substituents of R^{a} or the substituents of R^{b} do not form a ring), and hydrogen of the substituents may be replaced with deuterium.

2. The compound of claim 1, wherein Formula 1 is represented by one of Formula 1-1 to Formula 1-4: 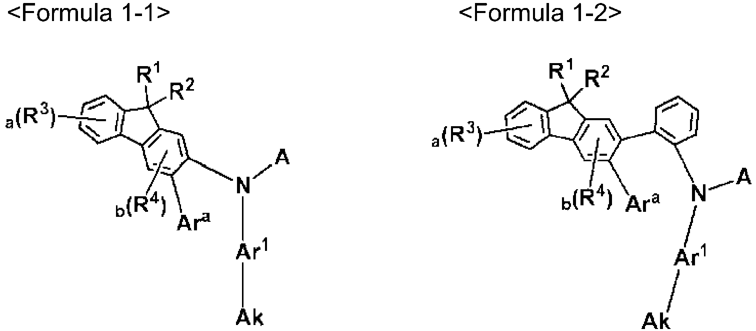 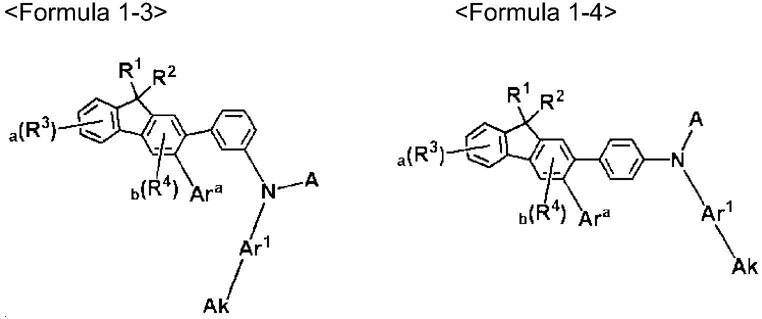 in Formula 1-1 to Formula 1-4, A, R¹ to R⁴, Ar¹, Ar^{a}, Ak, a and b are the same as defined in claim 1.

3. The compound of claim 1, wherein Formula A is represented by one of Formula A-1 to Formula A-4: 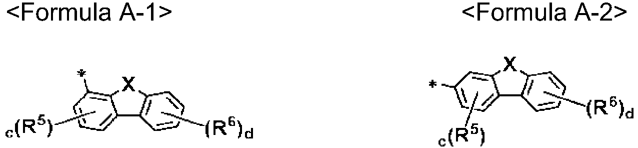 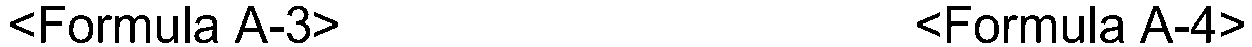 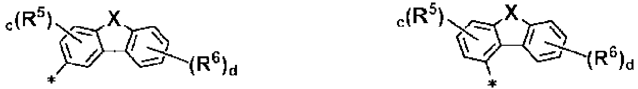 in Formula A-1 to Formula A-4, X, R⁵, R⁶, c and d are the same as defined in claim 1.

4. The compound of claim 1, wherein Ar¹ is selected from the group consisting of Formula L-1 to Formula L-27, and L¹ is a single bond or selected from the group consisting of Formula L-1 to Formula L-27: 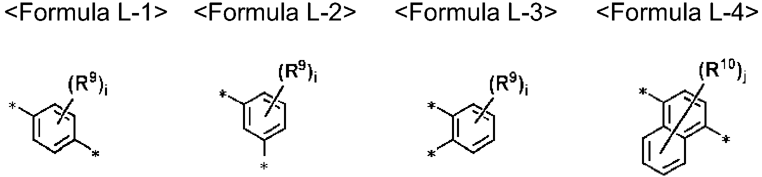 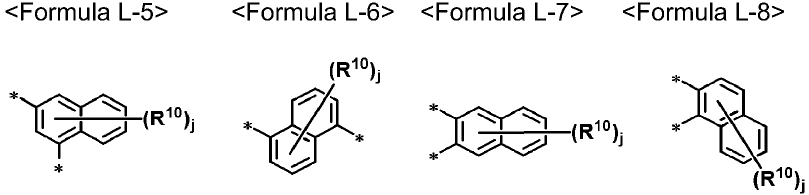 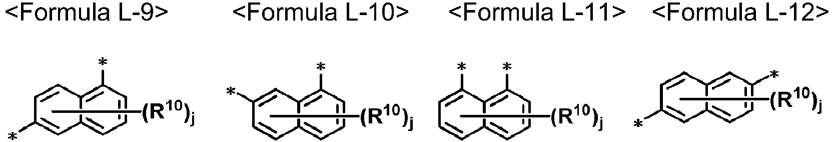 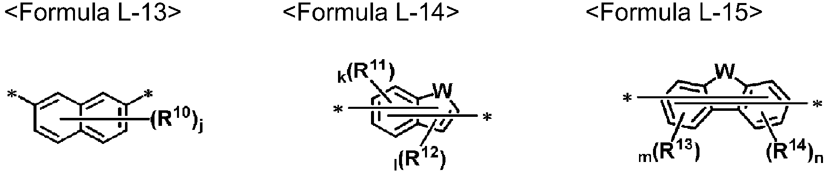 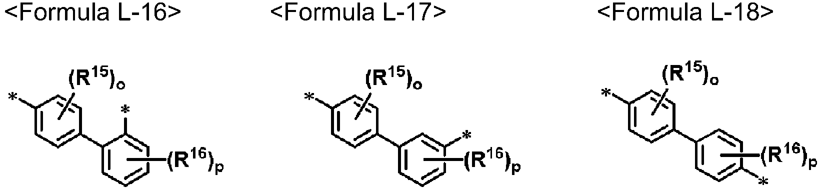 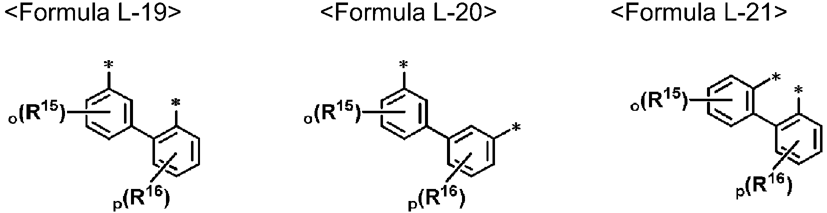 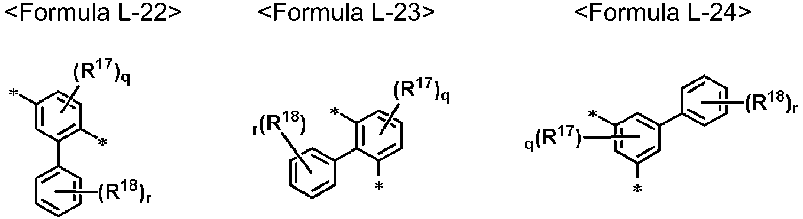 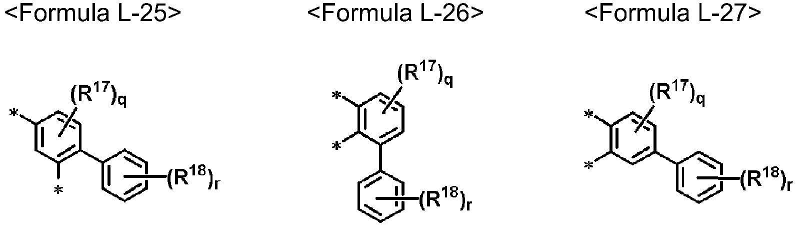 in Formula L-1 to Formula L-27,
W is O, S, C(R₁), C(R₂)(R₃), N or N(Ar₁), with the proviso that when L-14 and L-15 are connected via W, W is C(R₁) or N,
R⁹ to R¹⁸, R₁ to R₃ are each independently selected from the group consisting of hydrogen; deuterium; halogen; a silane group unsubstituted or substituted with a C₁-C₂₀ alkyl group or a C₆-C₂₀ aryl group; a phosphine oxide substituted or unsubstituted with a C₁-C₂₀ alkyl group or a C₆-C₂₀ aryl group; a cyano group; a nitro group; a C₁-C₂₀ alkylthio group; a C₁-C₂₀ alkoxy group; a C₆-C₃₀ aryloxy group; a C₆-C₃₀ arylthio group; a C₁-C₂₀ alkyl group; a C₂-C₂₀ alkenyl group; a C₂-C₂₀ alkynyl group; a C₆-C₃₀ aryl group; a deuterium-substituted C₆-C₃₀ aryl group; a C₃-C₃₀ alicyclic group; a fused ring of a C₆-C₃₀ aromatic ring and a C₃-C₃₀ alicyclic group; and a C₂-C₃₀ heterocyclic group comprising at least one heteroatom selected from the group consisting of O, N, S, Si and P, and adjacent groups may be bonded to each other to form a ring,
Ar₁ is selected from the group consisting of a C₁-C₂₀ alkyl group; a C₆-C₃₀ aryl group; a deuterium-substituted C₆-C₃₀ aryl group; a C₃-C₃₀ alicyclic group; a fused ring of a C₆-C₃₀ aromatic ring and a C₃-C₃₀ alicyclic group; and a C₂-C₃₀ heterocyclic group comprising at least one heteroatom selected from the group consisting of O, N, S, Si and P, and
i, k, m, n, o and p are each an integer of 0 to 4, j is an integer of 0 to 6, l is an integer of 0 to 2, q is an integer of 0 to 3, and r is an integer of 0 to 5.

5. The compound of claim 1, wherein the compound represented by Formula 1 is any one of the following compounds:

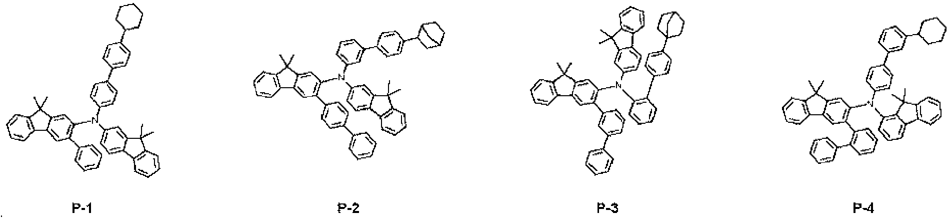

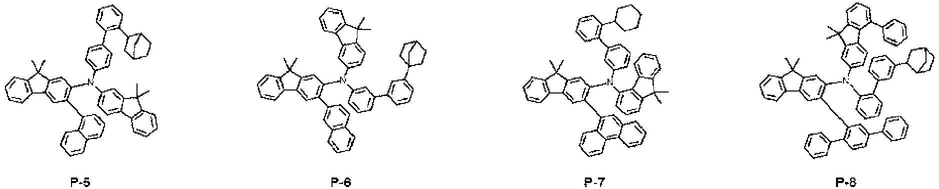

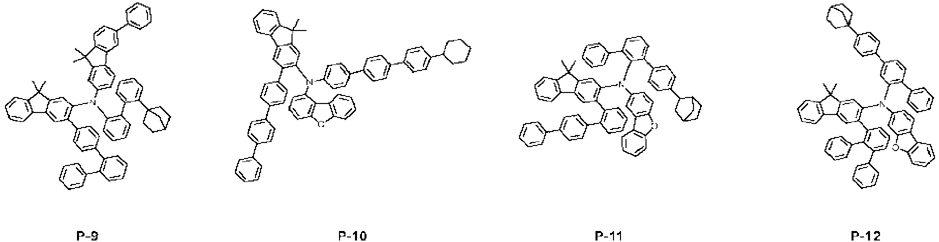

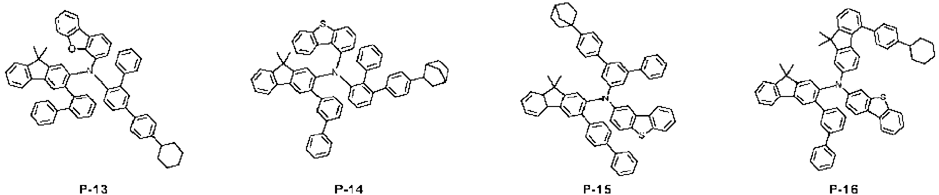

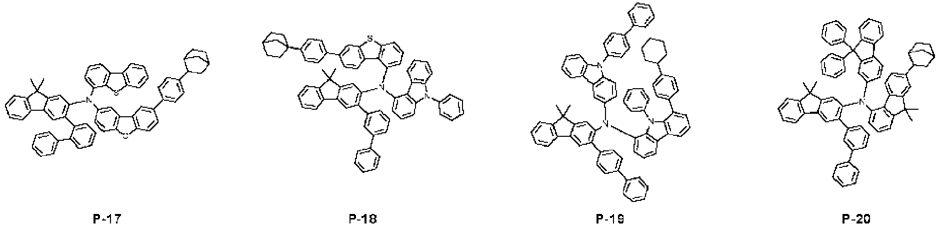

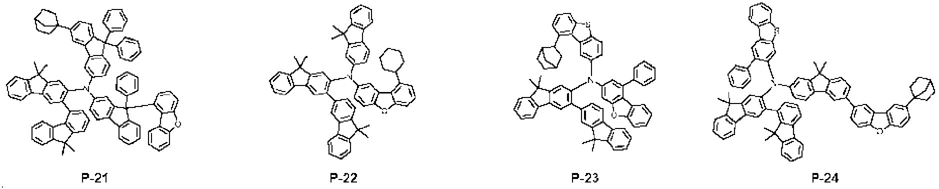

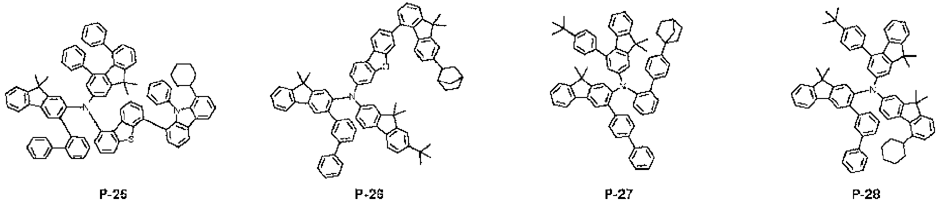

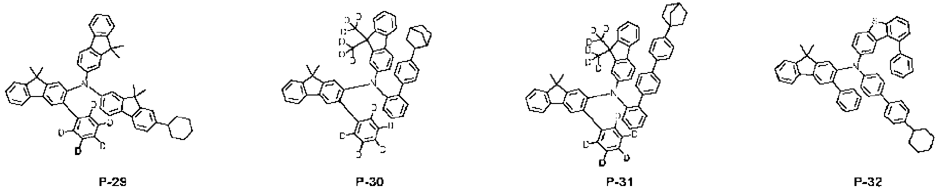

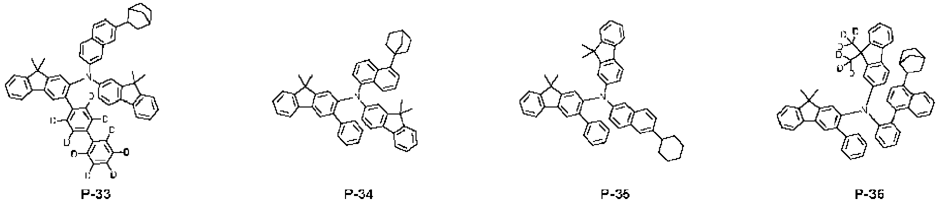

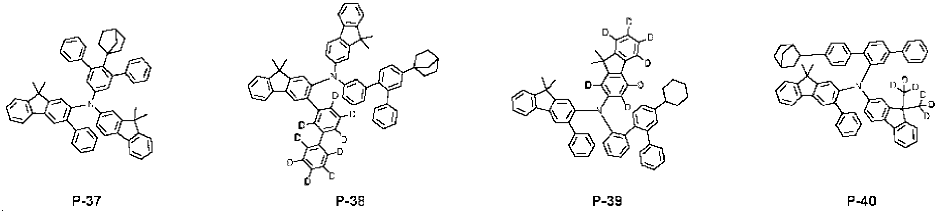

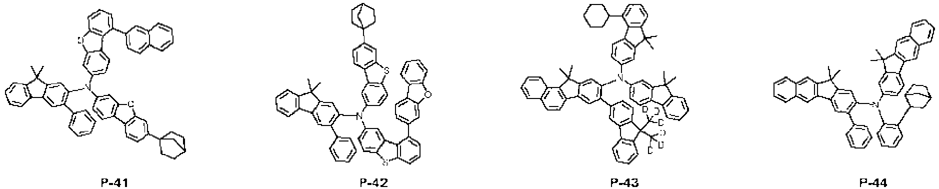

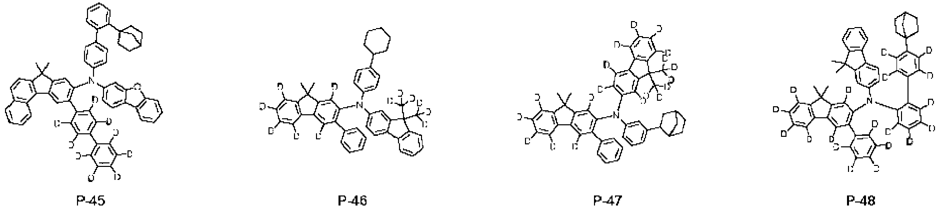

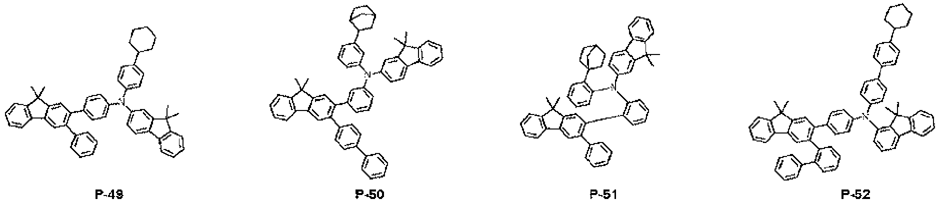

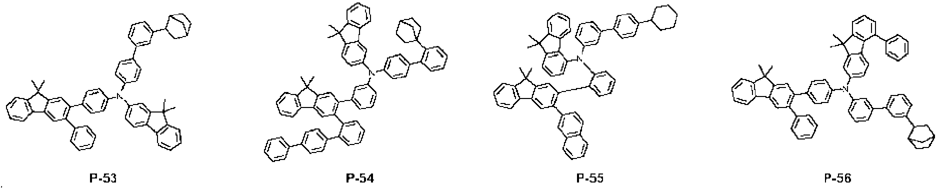

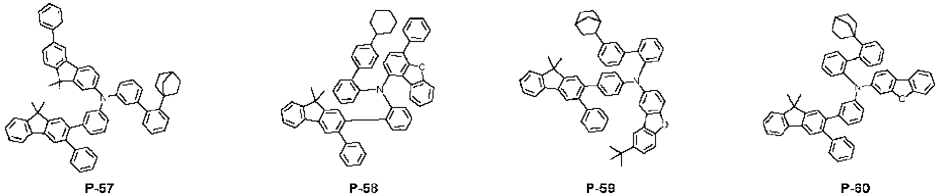

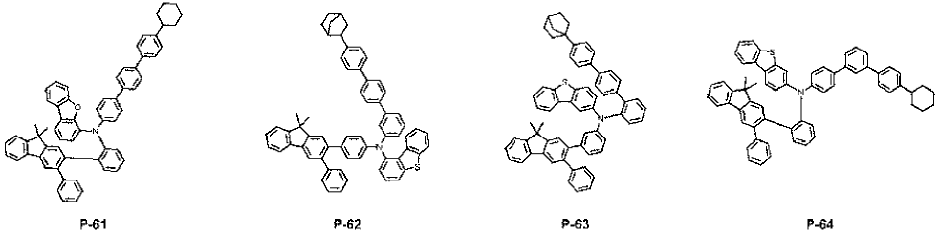

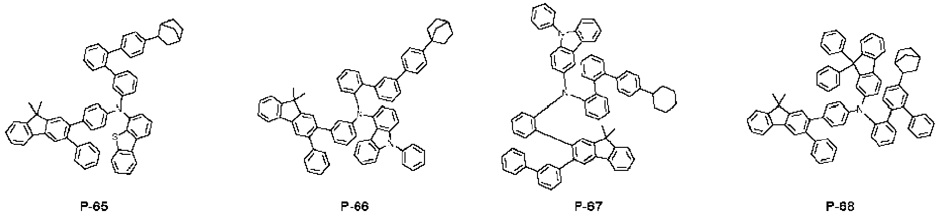

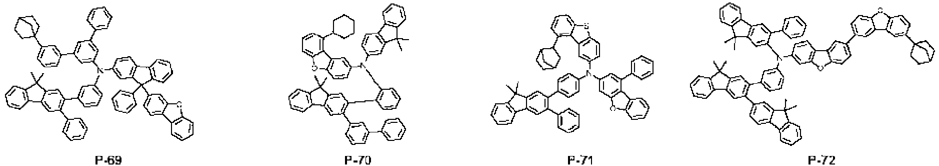

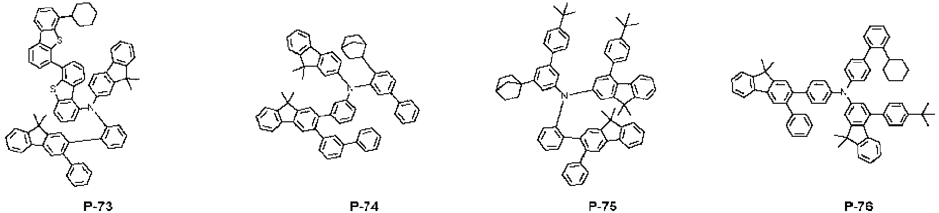

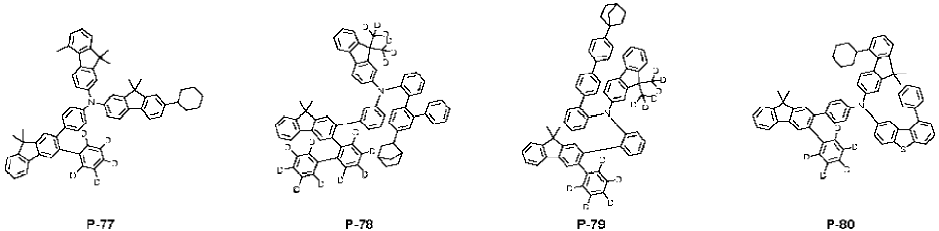

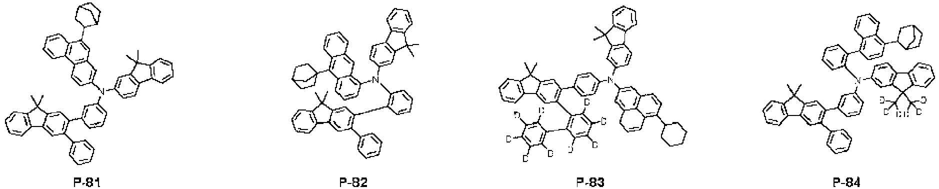

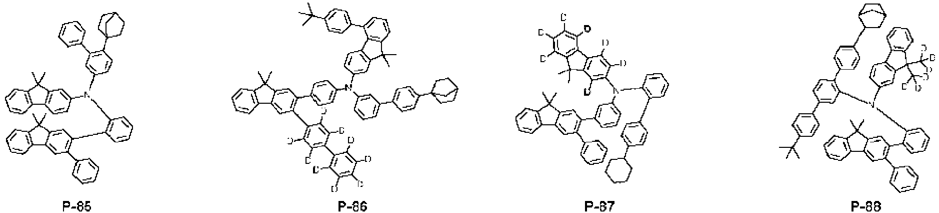

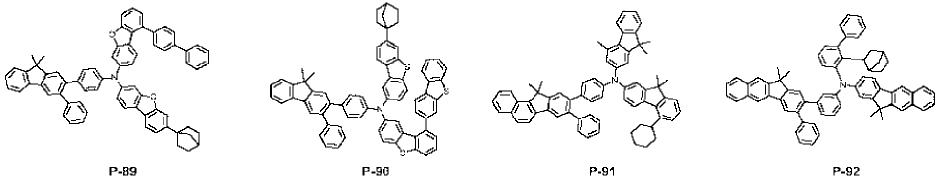

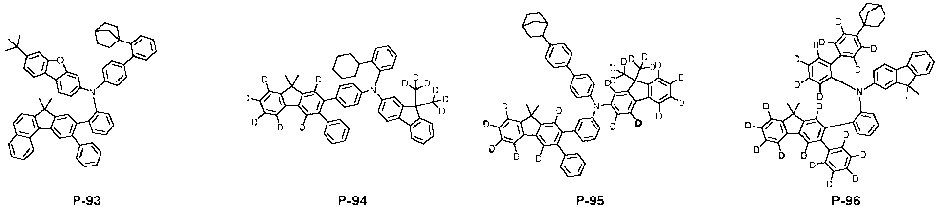

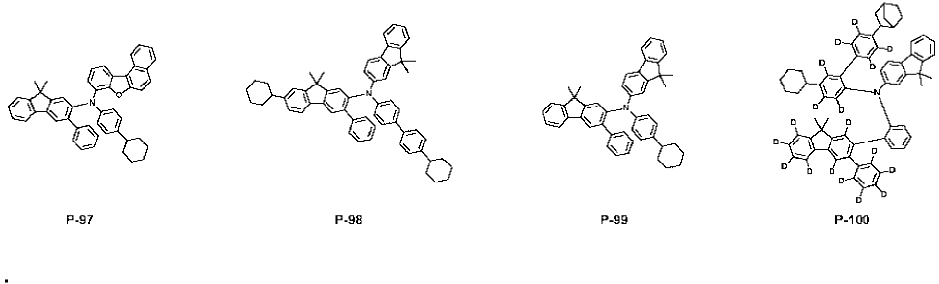

6. An organic electronic device comprising a first electrode, a second electrode, and an organic layer between the first electrode and the second electrode, wherein the organic layer comprises the compound of claim 1.

7. The organic electronic device of claim 6, wherein the organic layer comprises an emission auxiliary layer, and the emission auxiliary layer comprises the compound of claim 1.

8. The organic electronic device of claim 6, wherein the organic layer comprises two or more stacks, and the two or more stacks each comprise a hole transport layer, an emission layer and an electron transport layer formed sequentially on the first electrode.

9. An electronic apparatus comprising a display device and a control unit for driving the display device, wherein the display device comprises the organic electronic device of claim 6.

10. A method for recovering a compound represented by Formula 1 of claim 1, comprising:
depositing an organic layer material comprising a compound represented by Formula 1 using deposition equipment;
recovering the organic layer material attached to the deposition equipment; and
purifying the recovered organic layer material to obtain the compound represented by Formula 1 with a purity of 99.9% or higher.
